(19) **Europäisches Patentamt / European Patent Office / Office européen des brevets**

(11) **EP 3 766 418 B1**

(12) **EUROPEAN PATENT SPECIFICATION**

(45) Date of publication and mention
of the grant of the patent:
**04.01.2023 Bulletin 2023/01**

(21) Application number: **19767190.2**

(22) Date of filing: **14.03.2019**

(51) International Patent Classification (IPC):
***A61B 5/11*** *(2006.01)*     ***A61B 5/113*** *(2006.01)*

(52) Cooperative Patent Classification (CPC):
**A61B 5/6891; A61B 5/0816; A61B 5/1036;
A61B 5/1113; A61B 5/1115; A61B 5/113;**
A61B 5/7246; A61B 5/7282

(86) International application number:
**PCT/JP2019/010459**

(87) International publication number:
**WO 2019/177080 (19.09.2019 Gazette 2019/38)**

(54) **BODY MOTION DETERMINATION SYSTEM**

SYSTEM ZUR BESTIMMUNG DER KÖRPERBEWEGUNG

SYSTÈME DE DÉTERMINATION DE MOUVEMENT CORPOREL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.03.2018   JP 2018046885**

(43) Date of publication of application:
**20.01.2021 Bulletin 2021/03**

(73) Proprietor: **Minebea Mitsumi Inc.
Kitasaku-gun, Nagano 3890293 (JP)**

(72) Inventors:
 • **TODOROKI, Shinsuke
   Kitasaku-gun, Nagano 389-0293 (JP)**

 • **MASUDA, Shigemi
   Kitasaku-gun, Nagano 389-0293 (JP)**

(74) Representative: **Novagraaf Group
Chemin de l'Echo 3
1213 Onex/ Geneva (CH)**

(56) References cited:
| | |
|---|---|
| WO-A1-2013/054712 | WO-A1-2017/056476 |
| WO-A1-2019/026782 | JP-A- 2010 264 127 |
| JP-A- 2016 209 327 | JP-A- 2017 080 141 |
| JP-A- 2017 164 397 | US-A1- 2006 169 282 |

**Description**

TECHNICAL FIELD

The present invention relates to a body motion determining (determination) system for determining whether or not there is a body motion in a (human) subject on a bed on the basis of a detection value of a load detector.

BACKGROUND ART

For the sites of medical treatment and caregiving service, it is proposed to determine a state of a subject on the basis of such a load of the subject on a bed as detected by load detectors. In particular, for example, it is proposed to determine whether or not the subject has a body motion on the basis of the detected load.

Patent Literature 1 discloses a body motion level determining apparatus provided to determine a body motion level of a user on a bed in a step-up manner on the basis of detection values fed from load sensors arranged under the legs of the bed. Patent Literature 2 discloses a sleep determining apparatus and a turnover detecting apparatus which are capable of determining that there is a body motion in a person on a bed on the basis of detection results of load sensors arranged under the legs of the bed. Other systems for monitoring a subject on a bed are for example known from US 2006/0169282 A1, JP 2016-209327 A and WO 2017/056476 A1.

**Citation List**

  Patent Literature 1: Japanese Patent Application Laid-open No. 2014-195543
  Patent Literature 2: Japanese Patent No. 5998559 , specification

SUMMARY

**Technical Problem**

An object of the present invention is to provide a body motion determining system capable of determining whether or not a subject on a bed has a body motion with higher precision on the basis of detection values of load detectors.

**Solution to the Problem**

This object is solved by a body motion determining system according to claim 1 and a bed system according to claim 8. Examples thereof are detailed in the dependent claims. According to a first aspect of the present invention, there is provided a body motion determining system configured to determine whether or not a subject on a bed has a body motion, the system including:

  a plurality of load detectors each configured to detect a load of the subject on the bed;
  a respiratory waveform obtaining unit configured to obtain a respiratory waveform of the subject based on a temporal variation of the load of the subject detected by each of the plurality of load detectors; and
  a body motion determining unit configured to determine whether or not the subject has the body motion, based on a comparison between a first threshold value and a standard deviation of the temporal variation of the load of the subject detected by at least one of the plurality of load detectors,
  wherein the body motion determining unit is configured to compensate the standard deviation to be used in the comparison by an amplitude of the respiratory waveform.

In the body motion determining system according to the first aspect, the body motion determining unit may be configured to compensate the standard deviation to be used in the comparison by a latest amplitude of the respiratory waveform, the latest amplitude being obtained based on a latest peak of the respiratory waveform which is the latest at a point of time of determining whether or not the subject has a body motion, and a peak directly preceding the latest peak.

In the body motion determining system according to the first aspect, the body motion determining unit may be configured to compensate the standard deviation to be used in the comparison by an average value of the latest amplitude of the respiratory waveform and at least one amplitude of the respiratory waveform in a period directly preceding a point of time of obtaining the latest amplitude.

In the body motion determining system according to the first aspect, the body motion determining unit may be configured to continue, after a determination that the subject has the body motion, to compensate the standard deviation using the latest amplitude at a point of time of the determination that the subject has the body motion.

In the body motion determining system according to the first aspect, the compensating is performed by dividing the standard deviation by the amplitude of the respiratory waveform.

In the body motion determining system according to the first aspect, the plurality of load detectors may include a first load detector, a second load detector, a third load detector, and a fourth load detector; and the body motion determining unit may be configured to determine whether or not the subject has the body motion based on a comparison between the first threshold value and a simple average of $\sigma_1/A_{AVn}$, $\sigma_2/A_{AVn}$, $\sigma_3/A_{AVn}$, and $\sigma_4/A_{AVn}$, provided that $\sigma_1$ is a first standard deviation of the temporal variation of the load of the subject detected by the first detector, $\sigma_2$ is a second standard deviation of the temporal variation of the load of the subject detected by the second detector, $\sigma_3$ is a third standard deviation of the temporal variation of the load of the subject detected by the third detector, $\sigma_4$ is a fourth standard deviation of the temporal variation of the load of the subject detected by the fourth detector, and $A_{AVn}$ is the average value.

In the body motion determining system according to the first aspect, the body motion determining unit may be configured to determine a period during which the standard deviation is equal to or lower than a predetermined value as a resting period in which the subject merely performs a respiration, and compensate the standard deviation to be used in the comparison by the amplitude of the respiratory waveform in the resting period.

The body motion determining system according to the first aspect may further include: a non-negative-valued average calculating unit configured to calculate a non-negative-valued average of a detection value of at least one of the load detectors; and

a threshold value setting unit configured to set a second threshold value to be used to determine whether or not the subject has a body motion, based on the non-negative-valued average calculated in the resting period in which the subject merely performs the respiration,
wherein the body motion determining unit may be configured to perform a determination to determine whether or not the subject has the body motion based on a comparison between the second threshold value and the standard deviation, and then, after determining that the subject has no body motion by the determination based on the comparison between the second threshold value and the standard deviation, perform a determination to determine whether or not the subject has the body motion based on the comparison between the first threshold value and the standard deviation; and
the body motion determining unit may be configured to compensate the standard deviation to be used in the comparison with the first threshold value by the amplitude of the respiratory waveform obtained in a period which is determined, by the determination based on the comparison between the second threshold value and the standard deviation, as a period in which the subject has no body motion.

According to a second aspect of the present invention, there is provided a bed system including:

a bed; and
the body motion determining system according to the first aspect.

The body motion determining system of the present invention can determine whether or not the subject on the bed has a body motion with higher precision on the basis of the detection values of the load detectors.

BRIEF DESCRIPTION OF THE DRAWINGS

Fig. 1 is a block diagram depicting a configuration of a body motion determining system according to an embodiment of the present invention.
Fig. 2 is an illustrative view depicting an arrangement of load detectors for a bed.
Fig. 3 is a flow chart depicting a method for determining a body motion by using the body motion determining system.
Fig. 4 is a schematic graph depicting an aspect of variation in a load value detected by a load detector in both a resting period when a subject only respires and a period when the subject is performing a body motion.
Fig. 5(a) is an illustrative view conceptionally depicting an aspect where the center of gravity of the subject oscillates or vibrates in a body axis direction of the subject according to the respirations of the subject.
Fig. 5(b) is a graph depicting an example of a respiratory waveform drawn on the basis of the oscillation of the center of gravity of the subject according to the respirations of the subject.
Fig. 6 is an illustrative view for explaining an example of specific method for determining a body motion with a body motion determining unit.

Fig. 7 is a block diagram depicting a configuration of a biological state monitoring system according to a referential embodiment of the present invention.

Fig. 8 is a flow chart depicting a method for monitoring a biological state by using the biological state monitoring system according to the referential embodiment.

Fig. 9 is a block diagram depicting a specific configuration of a body motion determining unit provided in the biological state monitoring system according to the referential embodiment.

Fig. 10 is a flow chart depicting a procedure of a body motion determining step performed by the body motion determining unit provided in the biological state monitoring system according to the referential embodiment.

Figs. 11(a) and 11(b) are illustrative views for explaining a method for finding a non-negative-valued average over a predetermined period for such a detection value of a load detector as to vary due only to the respirations of the subject, wherein Fig. 11(a) is a graph schematically depicting an aspect of variation in the detection value before a non-negative valuing process is performed while Fig. 11(b) is a graph schematically depicting an aspect of variation in the detection value after the non-negative valuing process is performed.

Fig. 12 is a graph schematically depicting the magnitude of variation (standard deviation) in the detection value of the load detector due to the respirations of the subject and the magnitude of variation (standard deviation) in the detection value of the load detector due to a small body motion of the subject, with respect to each of a subject whose respiratory amplitude is small, a subject whose respiratory amplitude is about average, and a subject whose respiratory amplitude is large.

Fig. 13 is a block diagram depicting an overall configuration of a bed system according to a modified embodiment of the present invention.

## DESCRIPTION OF EMBODIMENT

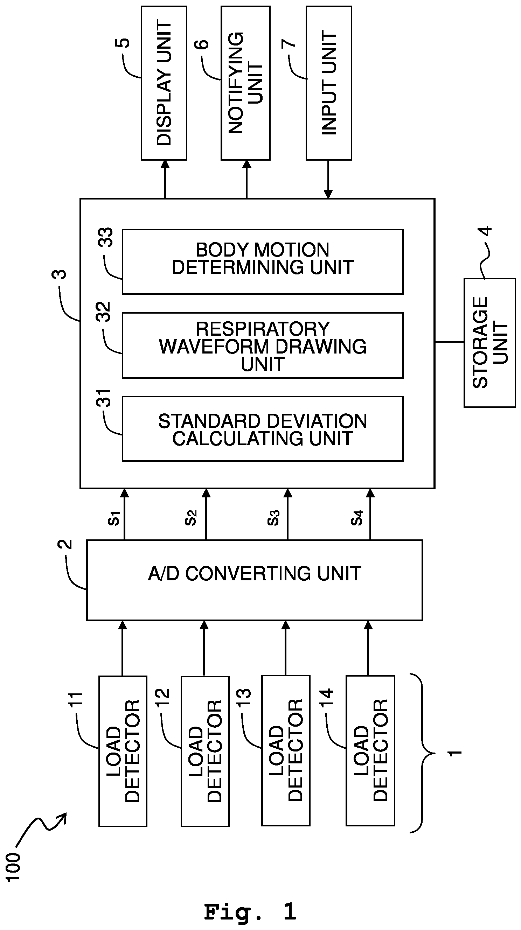

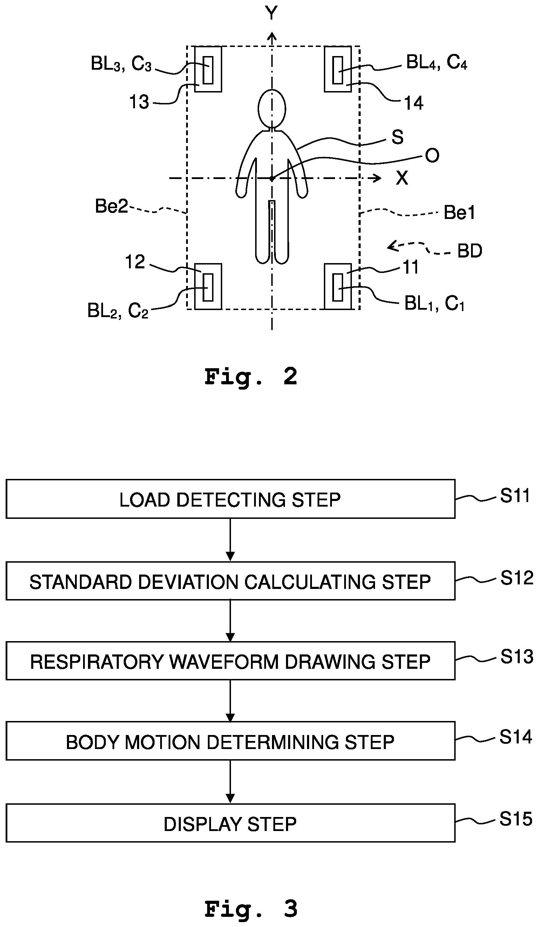

Explanations will be made on a body motion determining system 100 according to an embodiment of the present invention (Fig. 1), with an example of using the system together with a bed BD (Fig. 2) to determine whether or not there is a body motion in a subject S on the bed BD.

As shown in Fig. 1, the body motion determining system 100 of this embodiment primarily has a load detecting unit 1, a control unit 3, and a storage unit 4. The load detecting unit 1 and the control unit 3 are connected via an A/D converting unit 2. The control unit 3 is further connected to a display unit 5, a notifying unit 6, and an input unit 7.

The load detecting unit 1 includes four load detectors 11, 12, 13, and 14. Each of the load detectors 11, 12, 13, and 14 is a load detector for detecting a load by using, for example, a beam-type load cell. Such a load detector is disclosed, for example, in Japanese Patent No. 4829020 and Japanese Patent No. 4002905. Each of the load detectors 11, 12, 13, and 14 is connected to the A/D converting unit 2 by way of wiring or wirelessly.

As shown in Fig. 2, the four load detectors 11 to 14 of the load detecting unit 1 are arranged respectively under casters $C_1$, $C_2$, $C_3$, and $C_4$ fitted on the lower ends of legs $BL_1$, $BL_2$, $BL_3$, and $BL_4$ at the four corners of the bed BD used by the subject S.

The A/D converting unit 2 includes an A/D convertor to convert analog signals fed from the load detecting unit 1 to digital signals, and is connected respectively to the load detecting unit 1 and the control unit 3 by way of wiring or wirelessly.

The control unit 3 is a dedicated or general-purpose computer inside which a standard deviation calculating unit 31, a respiratory waveform drawing unit 32 (respiratory waveform calculating unit; respiratory waveform obtaining unit), and a body motion determining unit 33 are constructed.

The storage unit 4 is a storage device for storing data used in the body motion determining system 100 and, for example, a hard disk (magnetic disk) can be used for that purpose.

The display unit 5 is monitor such as a liquid crystal monitor or the like for displaying information outputted from the control unit 3 to users of the body motion determining system 100.

The notifying unit 6 includes a device, such as a speaker, for example, to auditorily perform a predetermined notification on the basis of the information fed from the control unit 3.

The input unit 7 is an interface for performing predetermined inputs for the control unit 3, which may be a keyboard and a mouse.

An explanation will be made on an operation of determining whether or not there is a body motion of the subject on the bed by using the body motion determining system 100 of such kind.

As depicted in the flow chart of Fig. 3, the determining of whether or not there is a body motion of the subject performed by using the body motion determining system 100 includes a load detecting step S11 for detecting the load of a subject S, a standard deviation calculating step S12 for calculating a standard deviation showing the degree of variation of the detected load, a respiratory waveform drawing step S13 for drawing a respiratory waveform of the subject on the basis of the detected load, a body motion determining step S14 for determining the body motion of the subject by using the standard deviation found in the standard deviation calculating step S12, and the amplitude of the respiratory waveform drawn in the respiratory waveform drawing step S13, and a display step S15 for displaying the result of determining the body motion.

[The load detecting step]

In the load detecting step S11, the load detectors 11, 12, 13, and 14 are used to detect the load of the subject S on the bed BD. The load of the subject S on the bed BD is applied dispersively to the load detectors 11 to 14 arranged respectively under the legs $BL_1$ to $BL_4$ of the bed BD at the four corners. The load of the subject S is detected dispersively by the four load detectors.

Each of the load detectors 11 to 14 detects the load (or variation of load), and outputs the detected load as an analog signal to the A/D converting unit 2. The A/D converting unit 2 converts the analog signal into a digital signal through at a sampling period (or cycle) of 5 milliseconds, for example, and then outputs the digital signal (to be referred to below as "load signal") to the control unit 3. Hereinafter, the term "load signals $s_1$, $s_2$, $s_3$, and $s_4$" will be used to refer respectively to the load signals obtained in the A/D converting unit 2 by converting the analog signals outputted from the load detectors 11, 12, 13, and 14 into the digital format.

[The standard deviation calculating step]

In the standard deviation calculating step S12, the standard deviation calculating unit 31 calculates standard deviations $\sigma_1$, $\sigma_2$, $\sigma_3$, and $\sigma_4$ (moving standard deviations) of a sampling value included in a predetermined sampling period (or time period) (5 seconds for example) for each of load signals $s_1$, $s_2$, $s_3$, and $s_4$. The calculation may be performed at any time (or continuously).

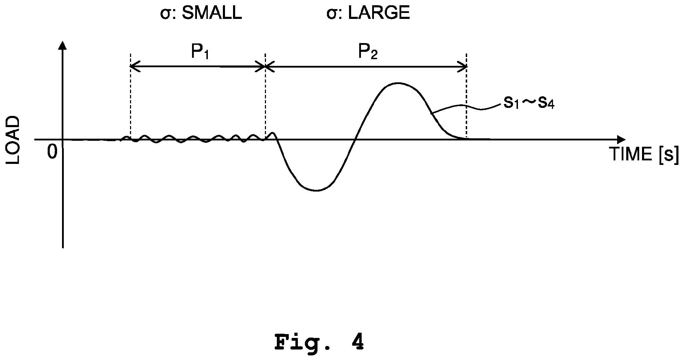

The standard deviation denotes the magnitude of variation(dispersion) of the sampling value. Thus, as depicted in Fig. 4, the standard deviations $\sigma_1$ to $\sigma_4$ become small during a period $P_1$ in which the subject S is resting on the bed BD and there is a small magnitude of variation in the load signals $s_1$ to $s_4$. On the other hand, the standard deviations $\sigma_1$ to $\sigma_4$ become large during a period $P_2$ in which the subject S is moving his/her body (in which there is a body motion arising in the subject S) and there is a large magnitude of variation in the load signals $s_1$ to $s_4$.

Therefore, during a period when there is a body motion arising in the subject S, the standard deviations $\sigma_1$ to $\sigma_4$ have larger values in comparison to a period when there is no body motion arising in the subject S.

In the present specification and in the present invention, the term "body motion" refers to any motion of the subject's head, torso (trunk, body-trunk), and/or four limbs. The body motion does not include motions of internal organs, blood vessels and the like along with the respirations, heartbeats, and the like. As an example, the body motion can be classified into a large body motion along with the motion of the subject S in the torso (trunk, body-trunk), and a small body motion along with the motion of the subject only in the four limbs, the head, and/or the like. One example of the large body motion is turn-over, sit-up or get-up, or the like, whereas one example of the small body motion is, a motion of the hands, the feet, the head or the like during sleep. When heartbeats, respirations and body motions arise in the subject, the load signals $s_1$ to $s_4$ fed from the load detectors 11 to 14 vary accordingly. The magnitude of variation increases in the order of the variation due to the heartbeats of the subject S, the variation due to the respirations of the subject S, the variation due to the small body motion of the subject S, and the variation due to the large body motion of the subject S.

Note that in the determination of a body motion of the subject described in the present specification and in the present invention, the magnitude of variation in the load signals $s_1$ to $s_4$ due to the heartbeats of the subject S is so small as neglectable. Therefore, in the present specification and in the present invention, the term "the subject only (merely) respires (or the subject merely performs a respiration)", the load values and the load signals "vary due only to the respiration(s)", and the like are used to mean that the subject has no body motion, and the load values and the load signals show no variation due to the body motion, but not to mean that the subject does not have heartbeats, the load values and the load signals do not include the variation due to the heartbeats, etc.

[The respiratory waveform drawing step]

In the respiratory waveform drawing step S13, the respiratory waveform drawing unit 32 (respiratory waveform calculating unit; respiratory waveform obtaining unit) draws a respiratory waveform of the subject S on the basis of the load signals $s_1$ to $s_4$.

The respiration of human is performed by moving the chest and the diaphragm to expand and shrink the lungs. In this context, when the air is inhaled (or an inspiration is performed), i.e., when the lungs are expanded, the diaphragm is lowered downwardly, and the internal organs are also moved downwardly. On the other hand, when the air is expired (or an expiration is performed), i.e., when the lungs are shrunk, the diaphragm is raised upwardly, and the internal organs are also moved upwardly. As disclosed in the specification of Japanese Patent No. 6105703 granted to the present applicant, the center of gravity G (of the subject) moves slightly along with the movement of the internal organs, the moving direction thereof being approximately along the subject's backbone extending direction (body axis direction).

In the present specification and in the present invention, the term "respiratory waveform" refers to a waveform showing an aspect of the oscillation of the subject's center of gravity oscillating in the subject's body axis direction due to the subject's respirations, by plotting the aspect on the temporal axis. One period of the respiratory waveform corresponds to one respiration of the subject (one inspiration and expiration). The amplitude of the respiratory waveform is affected by the subject's (physical) frame (build, physique) and/or respiratory depth. In particular, for example, if the subject has a large frame or the subject performs a deep respiration, then the amplitude becomes large, whereas if the subject has a small frame or the subject performs a shallow respiration, then the amplitude becomes small.

The respiratory waveform drawing unit 32 draws, in particular, the respiratory waveform in the following manner.

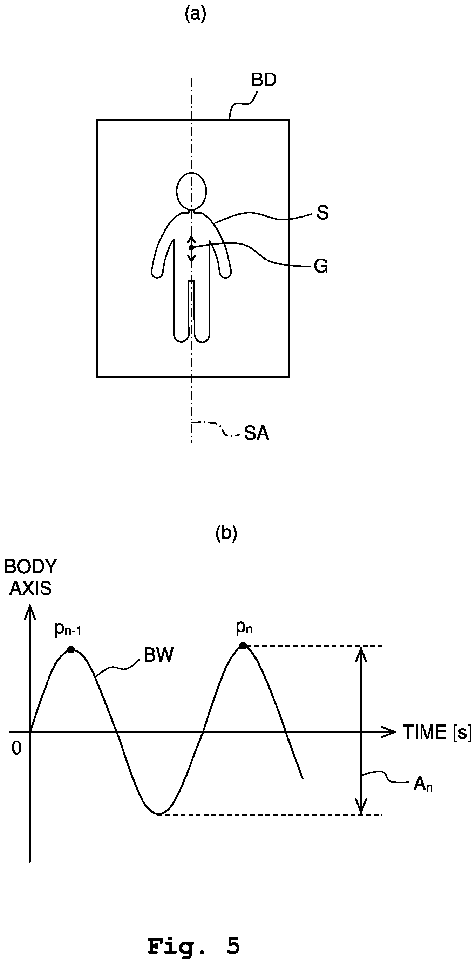

First, the respiratory waveform drawing unit 32 calculates the position of the center of gravity G of the subject S at each sampling time on the basis of the load signals $s_1$ to $s_4$ fed from the load detecting unit 1. As depicted in Fig. 5(a), the center of gravity G of the subject S oscillates in the direction of the body axis SA of the subject S in accordance with or due to the respiration of the subject S.

Next, the respiratory waveform drawing unit 32 draws a respiratory waveform BW (Fig. 5(b)) by way of plotting, on the vertical axis of a graph, the distance between the positions obtained by projecting the position of the center of gravity G at each time on the body axis SA, and the oscillation center of oscillation of the center of gravity G according to the respiration. The direction of the vertical axis of the graph matches the direction of the body axis SA. The horizontal axis of the graph shows time.

Note that it is not necessary for the respiratory waveform drawing unit 32 to actually draw the respiratory waveform but it is possible to only obtain data indicating the respiratory waveform.


[The body motion determining step]

In the body motion determining step S14, the body motion determining unit 33 uses the standard deviations $\sigma_1$ to $\sigma_4$ calculated in the standard deviation calculating step S12 and the amplitude of the respiratory waveform BW drawn in the respiratory waveform drawing step S13, to determine whether or not there is a body motion arising in the subject S.

The determination is performed in the following manner in particular for example.

First, the body motion determining unit 33 detects the peaks for the respiratory waveform BW drawn in the respiratory waveform drawing step S13, and calculates the difference between the minimum value over the period between the latest peak $p_n$ and the previous peak $p_{n-1}$, and the latest peak $p_n$, as the latest amplitude $A_n$ of the respiratory waveform BW (Fig. 5(b)). Then, the body motion determining unit 33 finds an average amplitude $A_{AVn}$ which is a simple average (value) of the amplitude $A_n$ and the amplitudes $A_{n-1}$, $A_{n-2}$, ... which are calculated before the amplitude $A_n$. While an arbitrary number of amplitudes $A_n$ can be used for calculating the average amplitude $A_{AVn}$, as one example, the number may be such as obtained in a sampling period of 5 seconds or so.

Note that in order to confirm that the respiratory waveform BW, which is involved in calculating the amplitudes $A_n$, $A_{n-1}$, $A_{n-2}$, ... and the amplitude $A_{AVn}$, is the respiratory waveform obtained from a resting period (during which the subject only respires without body motion arising), (i.e., in order to confirm that there is no error included due to the movement of the center of gravity because of some body motion), a comparison may be made between a threshold value $\sigma_0$ and either a simple average $\sigma_{Av}$ of the standard deviations $\sigma_1$ to $\sigma_4$ or any one of the standard deviations $\sigma_1$ to $\sigma_4$. The threshold value $\sigma_0$ is set at such a small value as able to reliably determine that there is no body motion arising in the subject S as long as the simple average $\sigma_{AV}$ or any one of the standard deviations $\sigma_1$ to $\sigma_4$ is smaller than the small value (conversely, even if the simple average $\sigma_{AV}$ or any one of the standard deviations $\sigma_1$ to $\sigma_4$ is not smaller than the small value, it is still possible that there is no body motion arising in the subject S).

Next, the body motion determining unit 33 finds normalized standard deviations $\sigma s_1$ to $\sigma s_4$ by the following (Formula 1).


[Formula 1]

$$\sigma s_m = \sigma_m / A_{AVn} \qquad (m = 1, 2, 3, 4)$$

Then, the body motion determining unit 33 finds a simple average $\sigma s_{AV}$ of the calculated normalized standard deviations $\sigma s_1$ to $\sigma s_4$ and, based on a comparison between the simple average $\sigma s_{AV}$ and a predetermined threshold value $\sigma s_{TH}$, determines whether or not there is a body motion arising in the subject S. In particular, for example, if the simple average $\sigma s_{AV}$ is larger than or equal to the threshold value $\sigma s_{TH}$, then it is determined that there is a body motion arising in the subject S, whereas if the simple average $\sigma s_{AV}$ is smaller than the threshold value $\sigma s_{TH}$, then it is determined that there is no body motion arising in the subject S.

During a period when it is determined that there is no body motion arising in the subject S, the body motion determining unit 33 calculates the latest amplitude $A_n$ for each one period of the respiratory waveform BW on the basis of the respiratory waveform BW obtained by the respiratory waveform drawing unit 32. Then, using the average amplitude $A_{AVn}$ calculated anew by using the value of the calculated latest amplitude $A_n$, and the values of the standard deviations $\sigma_1$ to $\sigma_4$ calculated at each sampling time (as an example, every 5 milliseconds), the body motion determining unit 33 calculates, successively, the normalized standard deviations $\sigma s_1$ to $\sigma s_4$ by the Formula 1, and calculates the simple average $\sigma s_{AV}$.

Determining whether or not there is a body motion arising in the subject S is performed by the successive comparisons between the predetermined threshold value $\sigma s_{TH}$ and the simple average $\sigma s_{AV}$ calculated successively in the above manner.

After it is determined as a result of the determination that there is a body motion arising in the subject S, the body motion determining unit 33 ceases to update the average amplitude $A_{AVn}$ and continues to calculate the simple average $\sigma s_{AV}$ using the average amplitude $A_{AVn}$ having been calculated by this point. Then, based on a comparison between the calculated simple average $\sigma s_{AV}$ and the threshold value $\sigma s_{TH}$, the body motion determining unit 33 continues to determine whether or not there is a body motion arising in the subject S. This is because during a period when there is a body motion arising in the subject S, the amplitude and period of the respiratory waveform BW vary greatly due to the influence of the body motion, and thereby it is difficult to calculate a new amplitude $A_n$ and the average amplitude $A_{AVn}$ without error.

Next, if it is determined again that there is no body motion arising in the subject S, then based on the respiratory waveform BW obtained by the respiratory waveform drawing unit 32, the body motion determining unit 33 calculates the value of the latest amplitude $A_n$ and, using the same, calculates the average amplitude $A_{AVn}$ anew. Then, the body motion determining unit 33 calculates the simple average $\sigma s_{AV}$ using the latest average amplitude $A_{AVn}$ and, based on a comparison between the calculated simple average $\sigma s_{AV}$ and the predetermined threshold value $\sigma s_{TH}$, the body motion determining unit 33 continues to determine whether or not there is a body motion arising in the subject S.

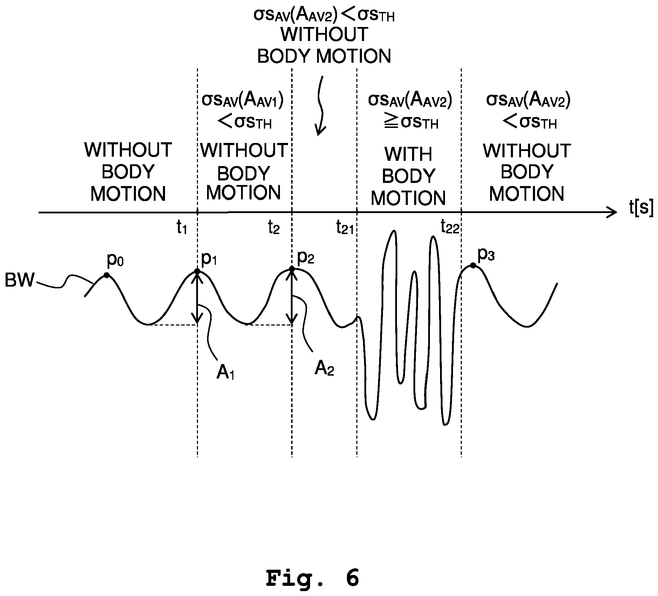

Referring to Fig. 6, a particular example of this step will be explained.

As depicted in Fig. 6, if a peak of the respiratory waveform BW and the latest amplitude $A_1$ are obtained at the time $t_1$, then for the period after the time $t_1$, the body motion determining unit 33 uses the average amplitude $A_{AV1}$ which is the simple average of the amplitude $A_1$ and the amplitudes $A_0$, $A_{-1}$, ... (all not shown) obtained right before the amplitude $A_1$, to successively calculate the normalized standard deviations $\sigma s_1$ to $\sigma s_4$ and the simple average $\sigma s_{AV}$ (to be referred to here as $\sigma s_{AV}$ ($A_{AV1}$)); and determines whether or not there is a body motion arising in the subject S based on a comparison between the simple average $\sigma s_{AV}$ ($A_{AV1}$) and the threshold value $\sigma s_{TH}$.

Next, if another peak $p_2$ of the respiratory waveform BW and the latest amplitude $A_2$ are obtained at the time $t_2$, then for the period after the time $t_2$, the body motion determining unit 33 uses the average amplitude $A_{AV2}$ calculated by using the amplitude $A_2$, to successively calculate the normalized standard deviations $\sigma s_1$ to $\sigma s_4$ and the simple average $\sigma s_{AV}$ (to be referred to here as $\sigma s_{AV}$ ($A_{AV2}$)); and determines whether or not there is a body motion arising in the subject S, based on the comparison between the simple average $\sigma s_{AV}$ ($A_{AV2}$) and the threshold value $\sigma s_{TH}$.

At the time $t_{21}$, if there is a body motion arising in the subject S, then the body motion determining unit 33 determines that there is a body motion arising in the subject S on the basis of the simple average $\sigma s_{AV}$ ($A_{AV2}$) being equal to or larger than the threshold value $\sigma s_{TH}$. Then, after that, for the period when the simple average $\sigma s_{AV}$ ($A_{AV2}$) is equal to or larger than the threshold value $\sigma s_{TH}$, the body motion determining unit 33 continues to determine the body motion using the simple average $\sigma s_{AV}$ ($A_{AV2}$).

At the time $t_{22}$, if the body motion of the subject S has come to an end, then the body motion determining unit 33 determines that there is no body motion arising in the subject S on the basis of the simple average $\sigma s_{AV}$ ($A_{AV2}$) being smaller than the threshold value $\sigma s_{TH}$. After that, the body motion determining unit 33 continues to determine the body motion using the simple average $\sigma s_{AV}$ ($A_{AV2}$) until the latest amplitude $A_n$ of the respiratory waveform BW is obtained and the latest average amplitude $A_{AVn}$ is calculated again.

The body motion determining unit 33 uses the amplitude $A_n$ (average amplitude $A_{AVn}$) of the respiratory waveform to normalize the values of the standard deviations $\sigma_1$ to $\sigma_4$. The reason therefor is as follows.

As described above, generally speaking, the values of the standard deviations $\sigma_1$ to $\sigma_4$ become larger during a period when there is a body motion arising in the subject S. Therefore, it is conceivable to determine whether or not there is a body motion arising in the subject S by way of comparison between the values of the standard deviations $\sigma_1$ to $\sigma_4$ and a predetermined threshold value.

However, according to the discovery and knowledge of the present inventors, for a subject who has a large frame (build, physique), the values of the standard deviations $\sigma_1$ to $\sigma_4$ may still be comparatively large even during a period of only performing respirations without the body motion because the movement of his/her internal organs due to the respirations is also large. Further, even for a subject who does not have a large frame, the values of the standard deviations $\sigma_1$ to $\sigma_4$ may still become large if, for example, the subject performs a deep respiration. Hence, if the body motion determination is performed by comparing the values of the standard deviations $\sigma_1$ to $\sigma_4$ to a predetermined threshold value, in those cases, even though there is no body motion arising in the subject, a mistaken determination may be made to give the incorrect result that there is a body motion arising in the subject.

On the other hand, if the respiratory waveform is focused on, then as described earlier on, the amplitude of the respiratory waveform is affected by the subject's frame and/or respiratory depth; therefore, if the subject has a large frame or the subject performs a deep respiration, then the amplitude becomes large, whereas if the subject has a small frame or the subject performs a shallow respiration, then the amplitude becomes small.

That is, by dividing the values of the standard deviations $\sigma_1$ to $\sigma_4$ by the average amplitude $A_{AVn}$ of the respiratory waveform BW to perform the normalization, it is possible to reduce (compensate; correct) the influence on the values of the standard deviations $\sigma_1$ to $\sigma_4$, exerted by the subject's frame and/or respiratory depth. Then, by using the normalized standard deviations $\sigma s_1$ to $\sigma s_4$ obtained through such normalization to determine the body motion, it is possible to raise the precision of determining the body motion.

[The display step]

In the display step S15, the control unit 3 causes the display unit 5 to display the determination result of the body motion determining step S14. Further, in the display step S15, the notifying unit 6 may be used to perform a notification in addition to or instead of the display using the display unit 5. In this case, for example, when the subject S has a body motion, a notification sound may be emitted to notify the nurses, caregivers and/or others who are the users of the body motion determining system 100 that a body motion is arising.

The effects of the body motion determining system according to this embodiment are summarized as follows.

The body motion determining system 100 of this embodiment determines the body motion by using the simple average $\sigma s_{AV}$ of the normalized standard deviations $\sigma s_1$ to $\sigma s_4$ which are compensated (corrected) by normalizing the values of the standard deviations $\sigma_1$ to $\sigma_4$ by the average amplitude $A_{AVn}$ of the respiratory waveform BW. Therefore, there are reduced influences exerted by the change in the respiratory depth and/or the physical description of the subject S, on the values of the standard deviations $\sigma_1$ to $\sigma_4$ and, consequently, on the body motion determination. Hence, the determination is made with high precision.

The body motion determining system 100 of this embodiment normalizes the values of the standard deviations $\sigma_1$ to $\sigma_4$ by using the average amplitude $A_{AVn}$, of the respiratory waveform BW, calculated on the basis of the respirations performed by the subject S during the period right before the point of time of determining the body motion of the subject S. In this manner, because the values of the standard deviations $\sigma_1$ to $\sigma_4$ are normalized with high precision, having reflected the actual respiratory condition of the subject S, the body motion determination is also made with high precision. Further, because the normalization is performed by the average amplitude $A_{AVn}$, even if any abnormal value arises in the amplitude $A_n$ of the respiratory waveform BW for some reason, the influence exerted by the abnormal value on the determination can still be reduced.

The body motion determining system 100 according to this embodiment uses the load detectors 11 to 14 arranged under the legs $BL_1$ to $BL_4$ of the bed BD to determine whether or not the subject S has a body motion. Therefore, it is not necessary to attach any measuring device to the body of the subject S so that the subject S will not feel discomfort and a sense of incongruity.

[Modified embodiments]

It is also possible to adopt the following modified embodiments with respect to the body motion determining system 100 according to the above embodiment.

In the body motion determining system 100 of the above embodiment, the standard deviations $\sigma_1$ to $\sigma_4$ are normalized by dividing the standard deviations $\sigma_1$ to $\sigma_4$ by the average amplitude $A_{AVn}$ to reduce (compensate; correct) the influence on the values of the standard deviations $\sigma_1$ to $\sigma_4$, exerted by the frame and/or respiratory depth of the subject S. However, the compensating (correcting) method by utilizing the amplitude of the respiratory waveform BW is not limited to that. In particular, for example, the standard deviations $\sigma_1$ to $\sigma_4$ may be normalized by way of dividing the same by the latest amplitude $A_n$ of the respiratory waveform BW.

Note that, it is also possible to compensate the threshold value by using the amplitude of the respiratory waveform BW and compare the standard deviations $\sigma_1$ to $\sigma_4$ to the compensated threshold value, instead of compensating the standard deviations $\sigma_1$ to $\sigma_4$ by using the amplitude of the respiratory waveform BW and then comparing the compensated values to the threshold value. In particular, for example, in the above embodiment, instead of dividing the standard deviations $\sigma_1$ to $\sigma_4$ by the average amplitude $A_{AVn}$, it is possible to perform a desired compensation (correction) by multiplying the predetermined threshold value by the average amplitude $A_{AVn}$. In this manner, the two methods mentioned above are practically equivalent and thus it is possible to appropriately select either the standard deviations or the threshold value used in the comparison for the compensation (correction) by the amplitude of the respiratory waveform. In the present specification the expression "compensating the standard deviations by the amplitude of the respiratory waveform" is termed to include compensating the threshold value by the amplitude of the respiratory waveform.

In the body motion determining system 100 of the above embodiment, the body motion determination is performed by the comparison between the simple average $\sigma s_{AV}$ of the normalized standard deviations $\sigma s_1$ to $\sigma s_4$ and the threshold value $\sigma s_{TH}$. However, it is also possible to determine the body motion by a comparison between at least one of the normalized standard deviations $\sigma s_1$ to $\sigma s_4$ and the threshold value, a comparison between simple average values of at least two or more of the normalized standard deviations $\sigma s_1$ to $\sigma s_4$ and the threshold value, a comparison between the total value of at least two or more of the normalized standard deviations $\sigma s_1$ to $\sigma s_4$ and the threshold value, or the like.

Further, in the body motion determining system 100 of the above embodiment, it is also possible to use a variance which is the squared standard deviation instead of the standard deviation. If the variance is normalized by the amplitude of the respiratory waveform, then the variance may be divided by the squared amplitude of the respiratory waveform. Therefore, in the present specification the standard deviation is termed to include the variance.

The body motion determining unit 33 may provide the threshold value used for the body motion determination with hysteresis. In particular, for example, with a first threshold value and a second threshold value larger than the first threshold value set in advance, under the condition that the subject S is determined as with no body motion arising therein, as far as the simple average $\sigma s_{AV}$ is yet smaller than the second threshold value, the subject S is not determined as with a body motion arising therein. On the other hand, under the condition that the subject S is determined as with a body motion arising therein, even if the simple average $\sigma s_{AV}$ is smaller than the second threshold value, the subject S is not determined as with no body motion arising therein, but is determined as with no body motion arising therein at the point of the simple average $\sigma s_{AV}$ becoming smaller than the first threshold value.

<Referential embodiment>

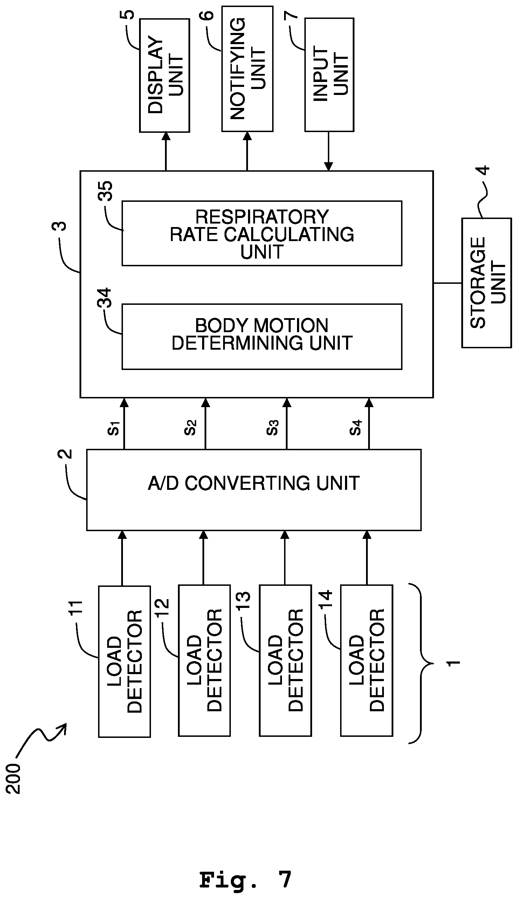

Explanations will be made on a body motion determining system according to a referential embodiment of the present invention, and on a biological state monitoring system 200 including the body motion determining system (Fig. 7), with an example of using the systems together with the bed BD (Fig. 2) to determine whether or not there is a body motion in the subject S on the bed BD, and to estimate the respiratory rate of the subject S.

In the following explanation, with the center of the rectangular bed BD (Fig. 2) as the center O, the axis X of the bed BD is defined as the axis extending in the breadthwise (widthwise) direction of the bed BD and passing through the center O, and the axis Y of the bed BD is defined as the axis extending in the lengthwise (longitudinal or up/down) direction of the bed BD and passing through the center O. In planar view of the bed BD, the positive side of the axis X is the right side of the center O of the bed BD whereas the negative side of the axis X is the left side thereof, and the positive side of the axis Y is the upper side of the center O of the bed BD whereas the negative side of the axis Y is the lower side thereof. When the subject S lies on the bed BD, generally he or she lies along the axis Y and the head is placed on the positive side and the feet are placed on the negative side in the axis-Y direction.

As shown in Fig. 7, the biological state monitoring system 200 of this referential embodiment is configured to construct a body motion determining unit 34 and a respiratory rate calculating unit 35 inside the control unit 3, instead of the standard deviation calculating unit 31, the respiratory waveform drawing unit 32, and the body motion determining unit 33. The other configurations are all the same as those of the body motion determining system 100 of the above embodiment.

Note that inside the biological state monitoring system 200 having the above configuration, the part excluding the respiratory rate calculating unit 35 of the control unit 3 corresponds to the body motion determining system of this referential embodiment.

An explanation will be made on an operation of monitoring the biological state (presence or absence of a body motion and the respiratory rate) of the subject on the bed by using the biological state monitoring system 200 of such kind.

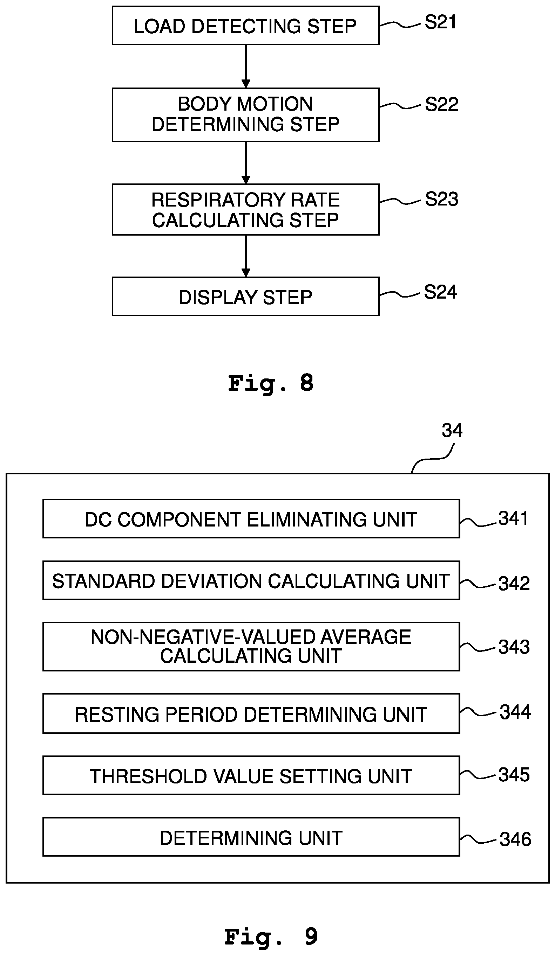

Monitoring the biological state of the subject by using the biological state monitoring system 200 includes: as depicted in the flow chart of Fig. 8, a load detecting step S21 for detecting the (body weight) load of the subject; a body motion determining step S22 for determining whether or not there is a body motion in the subject on the basis of the detected load; a respiratory rate calculating step S23 for calculating the respiratory rate of the subject with reference to whether or not there is a body motion in the subject; and a display step S24 for displaying the determination result of the body motion determining step S22 and/or the calculation result of the respiratory rate calculating step S23.

[The load detecting step]

The contents of the load detecting step S21 are the same as the contents of the load detecting step S11 performed in the body motion determining system 100 of the above embodiment.

[The body motion determining step]

In the body motion determining step S22, the body motion determining unit 34 uses at least one of the load signals $s_1$ to $s_4$, to determine whether or not there is a body motion in subject S.

As depicted in Fig. 9, the body motion determining unit 34 includes a DC (direct current) component eliminating unit 341, a standard deviation calculating unit 342, a non-negative-valued average calculating unit 343, a resting period determining unit 344, a threshold value setting unit 345, and a determining unit 346.

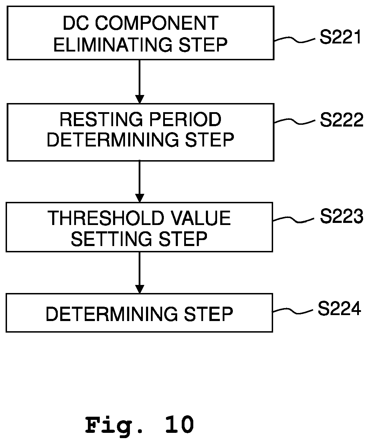

The body motion determining step S22 includes, as depicted in Fig. 10, a DC component eliminating step S221, a resting period determining step S222, a threshold value setting step S223, and a determining step S224.

In the body motion determining step S22, the body motion determining unit 34 first carries out the DC component eliminating step S221 using the DC component eliminating unit 341 to eliminate the DC component from each of the load signals $s_1$ to $s_4$. The DC components are eliminated, in particular for example, by finding a moving average over a predetermined period (15 seconds for example) for each of the load signals $s_1$ to $s_4$, and then eliminating the found moving average from the sampling value of each of the load signals $s_1$ to $s_4$.

Hereinbelow, the signals obtained after the DC components are eliminated from the load signals $s_1$ to $s_4$ will be referred to respectively as load signals $sc_1$ to $sc_4$.

The following steps, that is, the resting period determining step S222, the threshold value setting step S223 and the determining step S224, will be carried out using the load signals $sc_1$ to $sc_4$ obtained in the DC component eliminating step S221.

On the premise of carrying out the resting period determining step S222, the threshold value setting step S223 and the determining step S224, the body motion determining unit 34 constantly or continuously calculates a standard deviation σ with the standard deviation calculating unit 342, and constantly or continuously calculates a non-negative-valued average μ with the non-negative-valued average calculating unit 343.

The standard deviation calculating unit 342 constantly or continuously calculates standard deviations $\sigma_1$ to $\sigma_4$ of the sampling values included in a predetermined sampling period (time period) (5 seconds for example) for each of the load signals $sc_1$ to $sc_4$ obtained in the DC component eliminating step S221.

The standard deviations $\sigma_1$ to $\sigma_4$ calculated in the standard deviation calculating unit 342 are used to determine a resting period (a period in which the subject only respires without body motion) in the resting period determining step S222, and to determine whether or not the subject S has a body motion in the determining step S224 (to be described in detail later on).

The non-negative-valued average calculating unit 343 constantly or continuously calculates non-negative-valued averages $\mu_1$ to $\mu_4$ of sampling values $W_{11}$ to $W_{14}$ included respectively in the predetermined sampling period (time period) (5 seconds for example) for the respective load signals $sc_1$ to $sc_4$ obtained in the DC component eliminating step S221.

An explanation will be made about a method for calculating the non-negative-valued average with an exemplary case of calculating the non-negative-valued average $\mu_1$ over the sampling period of 5 seconds for the load signal $sc_1$ outputted in the resting period.

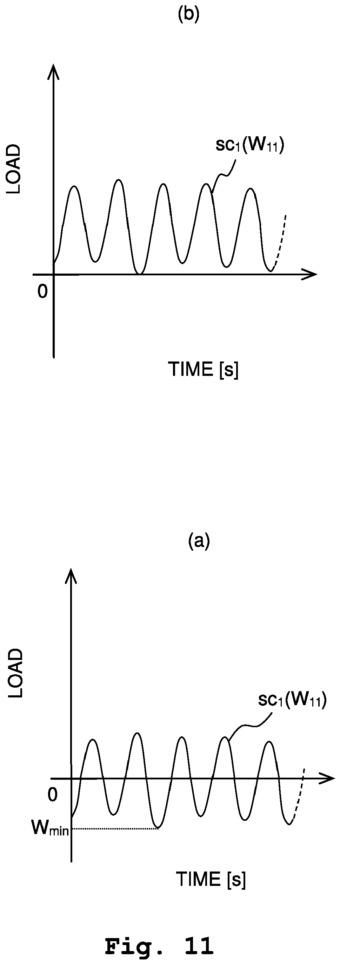

As depicted in Fig. 11(a), the load signal $sc_1$ oscillates or vibrates due only to the respirations of the subject S in the resting period (as described earlier on, the oscillating component due to the heartbeats is also included therein but is neglected for its smallness). In such load signal $sc_1$, the non-negative-valued average calculating unit 343 first specifies the minimal sampling value $W_{min}$ (negative) which is the minimum value of the sampling value $W_{11}$ included in that sampling period. Then, the non-negative-valued operation is made by subtracting the specified minimal sampling value $W_{min}$ from each sampling value $W_{11}$ within the sampling period (Fig. 11(b)).

Next, the non-negative-valued average calculating unit 343 calculates an average value of the respective sampling values $W_{11}$ having been non-negative-valued to acquire (obtain) the non-negative-valued average $\mu_1$. The non-negative-valued averages $\mu_2$ to $\mu_4$ are calculated in the same manner for the load signals $sc_2$ to $sc_4$.

The non-negative-valued averages $\mu_1$ to $\mu_4$ obtained in the resting period increase in value if the amplitude of load variation due to the respirations of the subject S increases, but decrease in value if the amplitude of load variation due to the respirations of the subject S decreases.

The non-negative-valued averages $\mu_1$ to $\mu_4$ calculated by the non-negative-valued average calculating unit 343 are used for setting a threshold value $\sigma_{th}$ in the threshold value setting step S223 (to be described in detail later on).

Returning to the flow chart of Fig. 10, the body motion determining unit 34 carries out the resting period determining step S222 with the resting period determining unit 344 when determining that a new subject S has arrived in the bed BD on the basis of an increase or the like of the load signals $s_1$ to $s_4$ (and/or the load signals $sc_1$ to $sc_4$), to determine that the subject S is situated in the resting period (the period when the subject only respires without body motion). This determination is carried out, in particular for example, by determining whether or not any of the standard deviations $\sigma_1$ to $\sigma_4$ calculated in the standard deviation calculating unit 342 is smaller than a predetermined threshold value $\sigma_0$ (which may be identical to the threshold value used in the body motion determining system 100 of the above embodiment).

Just as described above, the standard deviations $\sigma_1$ to $\sigma_4$ become small during the period when the load signals $sc_1$ to $sc_4$ vary little but become large during the period when the load signals $sc_1$ to $sc_4$ vary greatly. Therefore, any of the standard deviations $\sigma_1$ to $\sigma_4$ becomes small during a resting period (the period $P_1$ of Fig. 4, for example) when the subject S only respires without body motion. Hence, if the threshold value $\sigma_0$ is set to a sufficiently small value, then when any one of the standard deviations $\sigma_1$ to $\sigma_4$ reaches the threshold value $\sigma_0$, it is possible to determine that the subject S is (situated) in the resting period.

Note that as will be described later on, in the resting period, because there is difference among individuals in how small the variation due to the respirations will become in the load signals, depending on a specific subject, it is still possible to maintain the standard deviations $\sigma_1$ to $\sigma_4$ at larger values than the threshold value $\sigma_0$ even during the resting period when the subject only respires without body motion. In such a case, for example, with the standard deviations $\sigma_1$ to $\sigma_4$ being kept at certain values being a little larger than the threshold value $\sigma_0$ through a predetermined period (5 to 10 seconds, for example), it is possible to determine that the subject is in the resting period. Because respirations generally give a certain rhythm, if the standard deviations $\sigma_1$ to $\sigma_4$ have certain small values to some degree over a predetermined period, then even with those values being a little larger than the threshold value $\sigma_0$, there is still a high possibility of that period falling into the resting period.

If the resting period determining step S222 determines that the subject S is in the resting period, then the body motion determining unit 34 causes the threshold value setting unit 345 to perform the threshold value setting step S223.

In the threshold value setting step S223, the threshold value setting unit 345 estimates the magnitude of a respiratory amplitude of the subject S and, based on the estimated magnitude of the respiratory amplitude, sets the threshold value $\sigma_{th}$ to be used in the determining step S224.

The respiratory amplitude of the subject S refers to the amplitude of oscillation of the center of gravity G of the subject S due to the respirations of the subject S. That is, the respiratory amplitude is equal to the amplitude $A_n$ of the respiratory waveform BW calculated in the body motion determining system 100 of the above embodiment.

The threshold value setting unit 345 selects any one of the non-negative-valued averages $\mu_1$ to $\mu_4$ calculated by the non-negative-valued average calculating unit 343 in the resting period determined in the resting period determining step S222 and, according to the magnitude of the selected value, estimates the magnitude of the respiratory amplitude of the subject S. It can be said that if the non-negative-valued averages $\mu_1$ to $\mu_4$ in the resting period are large (or small), then the amplitudes of the oscillations of the load signals $s_1$ to $s_4$ and $sc_1$ to $sc_4$ due to the respirations of the subject S are large (or small), and thus, the respiratory amplitude of the subject S is also estimated as large (or small).

Next, the threshold value setting unit 345 sets the threshold value $\sigma_{th}$ to be used in the determining step S224 on the basis of the estimated magnitude of the respiratory amplitude of the subject S. In particular, for example, if the estimated magnitude of the respiratory amplitude is larger than a predetermined threshold value $\mu_{th}$, then the threshold value $\sigma_{th}$ is set to a first threshold value $\sigma_{th1}$, whereas if the estimated magnitude of the respiratory amplitude is smaller than the predetermined threshold value $\mu_{th}$, then the threshold value $\sigma_{th}$ is set to a second threshold value $\sigma_{th2}$ ($< \sigma_{th1}$). A description will be made later on about the reason of changing the magnitude of the threshold value $\sigma_{th}$ according to the magnitude of the respiratory amplitude of the subject S in this manner.

Note that without estimating the magnitude of the respiratory amplitude, any one of the calculated non-negative-valued averages $\mu_1$ to $\mu_4$ (a selected non-negative-valued average $\mu_s$) may be compared with the threshold value $\mu_{th}$. Then, if $\mu_s$ is larger than the threshold value $\mu_{th}$, then the threshold value $\sigma_{th}$ may be set to the first threshold value $\sigma_{th1}$, whereas if $\mu_s$ is smaller than the threshold value $\mu_{th}$, then the threshold value $\sigma_{th}$ may be set to the second threshold value $\sigma_{th2}$ ($< \sigma_{th1}$). In this case, practically, the threshold value $\sigma_{th}$ is also set on the basis of the magnitude of the respiratory amplitude of the subject S.

Thereafter, the body motion determining unit 34 uses the set threshold value $\sigma_{th}$ to perform the determining step S224 with the determining unit 346, so as to determine whether or not the subject S has a body motion. The body motion determining unit 34 is supposed to continuously use the threshold value $\sigma_{th}$ until the subject S leaves the bed BD and, thereafter, may perform the determining step S224 alone but not perform the resting period determining step S222 and the threshold value setting step S223.

Whether or not the subject S has a body motion is determined, in particular for example, by comparing the threshold value $\sigma_{th}$ set in the threshold value setting step S223 with any one of the standard deviations $\sigma_1$ to $\sigma_4$ (to be referred to below as "selected standard deviation $\sigma_{SE}$") of the load signals $sc_1$ to $sc_4$ calculated constantly or continuously by the standard deviation calculating unit 342.

During the period when the subject S has a body motion, the variation of the load signals $sc_1$ to $sc_4$ increases in magnitude, so that the standard deviations $\sigma_1$ to $\sigma_4$ and, furthermore, the selected standard deviation $\sigma_{SE}$ also increase (for example, during the period $P_2$ of Fig. 4). Therefore, the selected standard deviation $\sigma_{SE}$ is compared with the set threshold value $\sigma_{th}$ (that is, the $\sigma_{th1}$ or the $\sigma_{th2}$) to determine that the subject S has a body motion if the selected standard deviation $\sigma_{SE}$ becomes larger than the threshold value $\sigma_{th}$.

In this context, a description will be made as follows on the reason for the body motion determining unit 34 of this referential embodiment to set the threshold value $\sigma_{th}$ which differs according to each subject S by taking the magnitude of the respiratory amplitude of the subject S into referential consideration.

As described earlier on, the load signals $s_1$ to $s_4$ from the load detectors 11 to 14 vary according to the heartbeats, respirations and body motion of the subject S, and the magnitude of the oscillation changes according to the physical description of the subject S (such as the height, weight, body fat percentage, muscle mass, and the like). Then, according to the discovery and knowledge of the present inventors, there is a certain correlation between the magnitude of the load variation according to respirations (the magnitude of respiratory amplitude) and the magnitude of load variation according to the body motion.

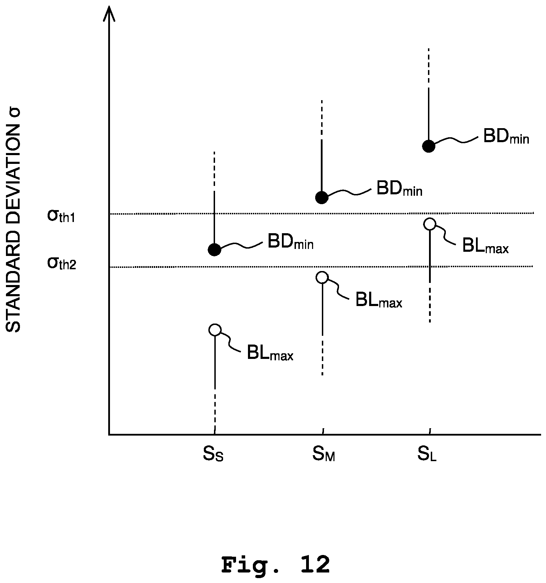

Fig. 12 is a graph schematically depicting such an aspect. In Fig. 12, the open circles show the upper limit values (the respiratory variation upper limit $BL_{max}$) of the magnitude of load variation showable according to the respirations of the subject S (shown in Fig. 12 with the standard deviation), while the filled circles show the lower limit values (the body motion variation lower limit $BL_{min}$) of the magnitude of load variation showable according to the body motion (small body motion) of the subject.

As depicted in Fig. 12, the respiratory variation upper limit $BL_{max}$ and the body motion variation lower limit $BD_{min}$ of a subject $S_S$ with a small respiratory amplitude are, respectively, smaller than the respiratory variation upper limit $BL_{max}$ and the body motion variation lower limit $BD_{min}$ of a subject $S_M$ with the average respiratory amplitude. On the other hand, the respiratory variation upper limit $BL_{max}$ and the body motion variation lower limit $BD_{min}$ of a subject $S_L$ with a large respiratory amplitude are, respectively, larger than the respiratory variation upper limit $BL_{max}$ and the body motion variation lower limit $BD_{min}$ of the subject $S_M$ with the average respiratory amplitude.

In this context, according to the discovery and knowledge of the present inventors, as shown in Fig. 12, the respiratory variation upper limit $BL_{max}$ of the subject $S_L$ with the large amplitude may become larger than the body motion variation lower limit $BD_{min}$ of the subject $S_S$ with the small respiratory amplitude. Therefore, it is difficult to use a single threshold value alone for determining the body motion of the subject $S_S$ with the small respiratory amplitude and for determining the body motion of the subject $S_L$ with the large respiratory amplitude, with high precision.

That is, suppose that the $\sigma_{th1}$ (Fig. 12) is used which is larger than the respiratory variation upper limit $BL_{max}$ of the subject $S_L$ but smaller than the body motion variation lower limit $BD_{min}$ of the subject $S_L$ so as to correctly determine the body motion of the subject $S_L$ with the large respiratory amplitude. Then, it is possible to correctly determine the body motion of the subject $S_L$ but, for the subject $S_S$ with the small respiratory amplitude, when the subject $S_S$ has a slight body motion, such a mistaken determination is liable to be made that there is no body motion in the subject $S_S$.

Conversely, suppose that the $\sigma_{th2}$ (Fig. 12) is used which is larger than the respiratory variation upper limit $BL_{max}$ of the subject $S_S$ but smaller than the body motion variation lower limit $BD_{min}$ of the subject $S_S$ so as to correctly determine the body motion of the subject $S_S$ with the small respiratory amplitude. Then, it is possible to correctly determine the body motion of the subject $S_S$ but, for the subject $S_L$ with the larger respiratory amplitude, when the subject $S_L$ only respires, such a mistaken determination is liable to be made that there is a body motion in the subject $S_L$.

In the biological state monitoring system 200 of this referential embodiment, however, based on the magnitude of the respiratory amplitude of the subject S, the threshold value $\sigma_{th}$ is set according to a specific subject S, and the set threshold value $\sigma_{th}$ is used to determine whether or not there is a body motion in the subject S. By virtue of this, it is possible to determine with high precision whether or not there is a body motion in each of a plurality of subjects S who differ in the magnitude of the respiratory amplitude.

[The respiratory rate calculating step]

In the respiratory rate calculating step S23, the respiratory rate calculating unit 35 calculates the respiratory rate of the subject S on the basis of at least one of the load signals $s_1$ to $s_4$.

In particular, for example, the respiratory rate calculating unit 35 calculates the respiratory rate of the subject S by carrying out the Fourier analysis on at least one of the load signals $s_1$ to $s_4$ (or the load signals $sc_1$ to $sc_4$) so as to specify the peak frequency coming up in the frequency band corresponding to the respirations (from about 0.2 Hz to about 0.33 Hz because the human respiration is performed about 12 to 20 times per minute). With the specified peak frequency, it is possible to calculate (estimate) the respiratory rate of the subject S over that period.

Further, in the period when the subject S has a body motion (the period $P_2$ of Fig. 4 for example), unlike the period when the subject S only respires (the period $P_1$ of Fig. 4 for example), the load signals $s_1$ to $s_4$ and $sc_1$ to $sc_4$ oscillates at a different frequency from the that of the oscillation due to the respirations of the subject S (or no oscillation is exhibited). Therefore, even if the Fourier analysis is applied to the load signals $s_1$ to $s_4$ and $sc_1$ to $sc_4$ obtained in such period, it is difficult to correctly calculate the respiratory rate of the subject S.

Therefore, the respiratory rate calculating unit 35 of this referential embodiment ceases to calculate the respiratory rate for the period when the subject S has a body motion on the basis of the determination result of the body motion determining unit 34. As for the respiratory rate of this period, an estimation value may be outputted on the basis of the respiratory rate of periods before and after that period, or a message may be outputted to indicate that the respiratory rate is unknown.

[The display step]

In the display step S24, the control unit 3 causes the display unit 5 to display the determination result of the body motion determining step S22 and/or the calculation result of the respiratory rate calculating step S23. Further, in the display step S24, the notifying unit 6 may be used to perform a notification in addition to or instead of the display using the display unit 5. In this case, for example, when the subject S has a body motion, a notification sound may be emitted to notify the nurses, caregivers and/or others who are the users of the biological state monitoring system 200 that a body motion is arising.

The effects of the body motion determining system and the biological state monitoring system 200 including the same according to this referential embodiment are summarized as follows.

In the body motion determining system and the biological state monitoring system 200 including the same according to this referential embodiment, the body motion determining unit 34 sets a suitable threshold value $\sigma_{th}$ for each subject S on the basis of the respiratory amplitude of the subject S, and uses the same to determine whether or not the subject S has a body motion. Hence, it is possible to determine whether or not there is a body motion with high precision for each of a plurality of subjects S who differ in the magnitude of the respiratory amplitude.

In the biological state monitoring system 200 of this referential embodiment, based on the determination result of the body motion determining unit 34, the respiratory rate calculating unit 35 excludes the periods difficult in calculating the respiratory rate of the subject S from the objects of calculating the respiratory rate. Therefore, there is a high reliability of the respiratory rate of the subject S calculated by the respiratory rate calculating unit 35.

The body motion determining system and the biologocal state monitoring system 200 including the same according to this referential embodiment use the load detectors 11 to 14 arranged under the legs $BL_1$ to $BL_4$ of the bed BD to monitor the biological state of the subject S. Therefore, it is not necessary to attach any measuring device to the body of the subject S so that the subject S will not feel discomfort and a sense of incongruity.

It is also possible to adopt the following modified embodiments with respect to the body motion determining system and the biological state monitoring system 200 according to the above referential embodiment.

In the body motion determining unit 34 of the above referential embodiment, whenever the determining unit 346 determines that the subject S has a body motion in the determining step S224, the resting period determining step S222 and the threshold value setting step S223 may be performed again to reset the threshold value $\sigma_{th}$ for the use in the determining step S224.

The magnitude of the respiratory amplitude of the subject S depends basically on the physical description of the subject S, and thus it does not change greatly during the monitoring period. Therefore, generally speaking, as far as the subject S is not changed, it is possible to use the threshold value $\sigma_{th}$, which is once set in the threshold value setting step S23, continuously in the successive monitoring.

However, patients, et al. who are receiving terminal cares (end-of-life medical treatment and end-of-life care) change often in physical condition as much as the respiratory amplitude changes, and such kind of change in physical condition often arises along with a body motion. Therefore, for the patients, et al. who are receiving terminal cares, it is possible to reset the threshold value $\sigma_{th}$ at each body motion and use the threshold value $\sigma_{th}$ according to the physical condition at the timing, so as to raise the precision of determining the body motion, and the reliability of the calculated respiratory rate.

On the other hand, the non-negative-valued average calculating unit 343 of the body motion determining unit 34 of the above referential embodiment may cease to calculate the non-negative-valued averages $\mu_1$ to $\mu_4$ after the threshold value $\sigma_{th}$ is set if the threshold value $\sigma_{th}$ once set is to be used continuously in the determining step S224. Alternatively, the non-negative-valued averages $\mu_1$ to $\mu_4$ may be calculated only in the case where the resting period determining unit 344 determines that the subject S is in the resting period in the resting period determining step S222.

In the above referential embodiment, the standard deviation calculating unit 342 of the body motion determining unit 34 may calculate variances $\sigma^2_1$ to $\sigma^2_4$ instead of or in addition to the standard deviations $\sigma_1$ to $\sigma_4$. In this case, in the threshold value setting step S223, a threshold value $\sigma^2_{th}$ is set according to the variances $\sigma^2_1$ to $\sigma^2_4$. Further, in the determining step S224, whether or not the subject S has a body motion is determined by comparing the threshold value $\sigma^2_{th}$ to any one or the sum of any two or more of the variances $\sigma^2_1$ to $\sigma^2_4$.

In the above referential embodiment, the body motion determining unit 34 compares whether or not any one of the standard deviations $\sigma_1$ to $\sigma_4$ is smaller than the predetermined threshold value $\sigma_0$, to determine that the subject S is in the resting period. However, without being limited to that, the body motion determining unit 34 may compare the sum of any two or more of the standard deviations $\sigma_1$ to $\sigma_4$ with the threshold value, to determine that the subject S is in the resting period.

In the above referential embodiment, the body motion determining unit 34 selects any one of the non-negative-valued averages $\mu_1$ to $\mu_4$ and, based on that one, estimates the magnitude of the respiratory amplitude of the subject S. However, without being limited to that, the body motion determining unit 34 may estimate the magnitude of the respiratory amplitude of the subject S on the basis of the sum of any two or more of the non-negative-valued averages

$\mu_1$ to $\mu_4$. For example, by using the summation of the non-negative-valued averages $\mu_1$ to $\mu_4$, it is possible to estimate the magnitude of the respiratory amplitude of the subject S with high precision regardless of the position of the subject S on the bed BD.

In the above referential embodiment, the body motion determining unit 34 sets the threshold value $\sigma_{th}$ to either the first threshold value $\sigma_{th1}$ or the smaller second threshold value $\sigma_{th2}$. However, without being limited to that, the body motion determining unit 34 may set the threshold value $\sigma_{th}$ in a more detailed manner according to the magnitude of the respiratory amplitude of the subject S estimated from the non-negative-valued averages $\mu_1$ to $\mu_4$. In particular, the threshold value $\sigma_{th}$ may be set to any of the first threshold value $\sigma_{th1}$ to an nth threshold value $\sigma_{thn}$ (n is a natural number larger than two) which are different from one another.

In the above referential embodiment, the body motion determining unit 34 compares the set threshold value $\sigma_{th}$ to the selected standard deviation $\sigma_{SE}$ which is any one of the standard deviations $\sigma_1$ to $\sigma_4$, to determine whether or not the subject S has a body motion. However, without being limited to that, the body motion determining unit 34 may determine whether or not the subject S has a body motion by comparing the threshold value $\sigma_{th}$ with the sum of any two or more of the standard deviations $\sigma_1$ to $\sigma_4$. Note that in this case, the threshold value $\sigma_{th}$ set in the threshold value setting step S223 is supposed to also accord to the contents of the standard deviations used in the determining step S224.

In the above referential embodiment, the body motion determining unit 34 may include a center of gravity position calculating unit instead of or in addition to the standard deviation calculating unit 342. The center of gravity position calculating unit uses sampling values $W_{11}$ to $W_{14}$ of the load signals $s_1$ to $s_4$ (or the load signals $sc_1$ to $sc_4$) fed from the load detectors 11 to 14, to calculate the position (X, Y) of the center of gravity G of the subject S.

The calculation of the position (X, Y) of the center of gravity G is performed in accordance with the following operation. Coordinates X-Y are set up for the bed BD as depicted in Fig. 2, and the coordinates of the load detectors 11, 12, 13, and 14 are supposed to be: $(X_{11}, Y_{11})$, $(X_{12}, Y_{12})$, $(X_{13}, Y_{13})$, and $(X_{14}, Y_{14})$, respectively. Then, the position G (X, Y) of the load applied on the bed BD is calculated with the following formulas.

(Formula 2)

$$X = \frac{X_{11} \times W_{11} + X_{12} \times W_{12} + X_{13} \times W_{13} + X_{14} \times W_{14}}{W_{11} + W_{12} + W_{13} + W_{14}}$$

$$Y = \frac{Y_{11} \times W_{11} + Y_{12} \times W_{12} + Y_{13} \times W_{13} + Y_{14} \times W_{14}}{W_{11} + W_{12} + W_{13} + W_{14}}$$

The center of gravity position calculating unit finds a temporal variation of the position (X, Y) of the center of gravity G, i.e., a center of gravity locus GT while calculating the position (X, Y) of the center of gravity G at a predetermined sampling period T on the basis of the formulas 2 and 3 given above. The acquired center of gravity locus GT is stored, for example, in the storage unit 4.

Here, the movement of the center of gravity G of the subject S is characterized as follows.

As described earlier on, the center of gravity G of the subject S oscillates in the direction of the body axis SA of the subject S according to the respirations of the subject S (Fig. 5(a)). Further, the center of gravity G of the subject S moves according to that if the subject S has a small body motion or a large body motion. Then, the moving distance of the center of gravity G over a predetermined period increases in the ascending order of the period when the subject S only respires, the period when the subject S has a small body motion, and the subject S has a large body motion.

Therefore, the body motion determining unit 34 can determine whether or not the subject S has a body motion by comparing a predetermined threshold value with the moving distance of the center of gravity G over a predetermined period. In particular, for example, it is possible to determine that the subject S has a small body motion if the moving distance D of the center of gravity G over a predetermined period is longer than a threshold value $D_{th}$.

In this modified embodiment, too, the body motion determining unit 34 causes the threshold value setting unit 345 to perform the threshold value setting step S223 to set the threshold value $D_{th}$ (for example, either a $D_{th1}$ or a smaller $D_{th2}$) according to the magnitude of the respiratory amplitude of the subject S.

In the above referential embodiment, the respiratory rate calculating unit 35 calculates the respiratory rate of the subject S by performing the Fourier transform on at least one of the load signals $s_1$ to $s_4$ (or the load signals $sc_1$ to

$sc_4$). However, the present invention is not limited to that.

In particular, for example, if the body motion determining unit 34 has the center of gravity position calculating unit, then it is possible to draw a respiratory waveform BW of the subject S (Fig. 5(b)) from the calculated position of the center of gravity G. The respiratory rate calculating unit 35 can regard the oscillation rate of the respiratory waveform BW drawn in this manner as the respiratory rate of the subject S.

Note that in this case, too, when the subject S has a body motion, the center of gravity G of the subject S moves greatly to deviate from the oscillation due to the respirations, so that the respiratory waveform BW also departs from the state of being oscillating due only to the respirations. Therefore, when the body motion determining unit 34 determines that the subject S has a body motion, calculation of the respiratory rate is ceased.

In the above referential embodiment, the control unit 3 may display the drawn respiratory waveform BW on the display unit 5.

In the above referential embodiment, the body motion determining unit 34 first determines that the subject S is in the resting period in the resting period determining step S222, and then uses the non-negative-valued average $\mu$ in that period to estimate the magnitude of the respiratory amplitude of the subject S. However, the present invention is not limited to that.

The subject S rarely moves constantly on the bed BD (to give rise to a body motion) but, usually, the subject S is resting without body motion (only respiring) during the most part of the period of his/her being present on the bed BD. Therefore, if the non-negative-valued averages $\mu_1$ to $\mu_4$ are observed continuously over a predetermined period of time (one minute or longer for example) without determining the resting period, then the minimal value of any one of (or the sum of any two or more of) the non-negative-valued averages $\mu_1$ to $\mu_4$ obtained in that period over time is often practically a value obtainable in the resting period (in other words, although the resting period is not distinctly determined, at the point when that minimal value is obtained, there is often a case where the subject is in the resting state, and the minimal value is the non-negative-valued average in the resting period). In this manner, the body motion determining unit 34 may estimate the magnitude of the respiratory amplitude of the subject S on the basis of the minimal value of the non-negative-valued averages obtained sequentially in the predetermined period of time.

Further, in this case, if such a value is observed as even smaller than the minimal value of the non-negative-valued averages once obtained, then on the basis of this value, the magnitude of the respiratory amplitude of the subject S may be estimated anew and/or the threshold value $\sigma_{th}$ be set anew as necessary.

Alternatively, it is possible to carry out the Fourier analysis on the load signals $s_1$ to $s_4$ (or the load signals $sc_1$ to $sc_4$) fed from the load detectors $s_{11}$ to $s_{14}$ to extract only the signals of the respiration band, and to estimate the magnitude of the respiratory amplitude of the subject S from the non-negative-valued average calculated on the basis of the extracted signals. According to this method, because it is possible to exclude the influence of body motion on the load signals $s_1$ to $s_4$ (or the load signals $sc_1$ to $sc_4$) by way of the Fourier analysis, the magnitude of the respiratory amplitude of the subject S can be estimated at any time without determining the resting period.

In the above referential embodiment and modified embodiments, as an example of the method for estimating the magnitude of the respiratory amplitude of the subject S on the basis of the non-negative-valued average $\mu$, it is possible to regard the same magnitude or predetermined multiples of the magnitude of the non-negative-valued average $\mu$ as the magnitude of the respiratory amplitude of the subject S.

In the above referential embodiment, the body motion determining unit 34 can also find the respiratory amplitude of the subject S without using the non-negative-valued average u.

In particular, for example, if there is a center of gravity position calculating unit and the respiratory waveform BW is drawn, then it is possible to estimate (determine) the respiratory amplitude of the subject S from the amplitude of the respiratory waveform BW. Alternatively, it is possible to estimate (determine) the respiratory amplitude of the subject S on the basis of at least one of the amplitudes of the load signals $s_1$ to $s_4$ and the load signals $sc_1$ to $sc_4$ in the resting period.

The biological state monitoring system 200 of the above referential embodiment may further include a biological state determining unit to determine other biological states than whether or not the subject S has a body motion and the respiratory rate of the subject S. Such a biological state determining unit determines, for example, whether the subject S is present in or absent from the bed, asleep or awake, dead or alive, etc., on the basis of whether or not the subject S has a body motion, the respiratory rate of the subject S, etc.

Because the biological state monitoring system 200 of the above referential embodiment can determine the body motion of the subject S and estimate the respiratory rate of the subject S with high precision, the biological state monitoring system 200 of the modified embodiments based on the former can also determine whether the subject S is present in or absent from the bed, asleep or awake, dead or alive, etc., with high precision.

The body motion determining system 100 of the above embodiment does not need to include all of the load detectors 11 to 14 but may include only any one of the four. For example, if there are three such load detectors, then it is still possible to detect the position of the center of gravity G of the subject S on the plane of the bed BD provided that the three load detectors are not arranged on a straight line. Further, the load detectors do not need to be arranged at the four corners of the bed but may be arranged in any positions as far as they can detect the load of the subject on the bed and the variation thereof. Further, the load detectors 11 to 14 are not limited to load sensors using beam-type load cells but, for example, force sensors are also usable.

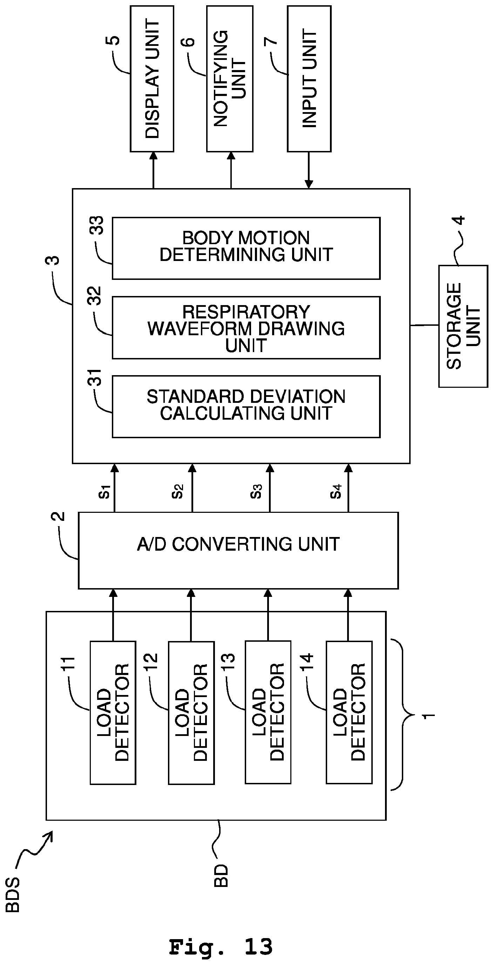

In the body motion determining system 100 of the above embodiment, the load detectors 11 to 14 are arranged respectively on the undersides of the casters C attached to the lower ends of the legs of the bed BD. However, there is no limitation thereto. Each of the load detectors 11 to 14 may be provided respectively between one of the four legs of the bed BD and the board of the bed BD. Alternatively, if each of the four legs of the bed BD can be divided into upper and lower portions, each of the load detectors 11 to 14 may be provided between the upper leg and the lower leg. Further alternatively, the load detectors 11 to 14 may be formed integral with or removable from the bed BD to construct a bed system BDS comprising the bed BD, and the body motion determining system 100 of this embodiment (Fig. 13).

In the body motion determining system 100 of the above embodiment, between the load detecting unit 1 and the A/D converting unit 2, it is possible to provide a signal amplifying unit to amplify the load signals fed from the load detecting unit 1, and a filtering unit to eliminate the noises from the load signals.

In the body motion determining system 100 of the above embodiment, the display unit 5 may include a printer for printing out the information indicating whether or not there is a body motion, a simplified visual display means such as lamps indicating whether or not there is a body motion, and/or the like, instead of the monitor or in addition to the monitor. Further, the notifying unit 6 may include a vibration generating unit for performing the notification by way of vibration, instead of the speaker or in addition to the speaker.

It is also possible to construct a body motion determining system by use of a combination of the body motion determining system 100 of the above embodiment and the body motion determining system of the above referential embodiment. Such body motion determining system, in particular for example, determines the body motion with the body motion determining system of the above referential embodiment without depending on the respiratory waveform BW for periods after the system is started up in which it is not possible to reliably draw the respiratory waveform BW and to calculate the average amplitude $A_{AVn}$. Then, when coming to the point of being able to reliably draw the respiratory waveform BW and calculate the average amplitude $A_{AVn}$, the body motion determining system 100 of the above embodiment starts to determine the body motion. For example, the body motion determining unit of such body motion determining system determines the body motion of the subject S in the same manner as the body motion determining unit 34 of the above referential embodiment, by using the threshold value $\sigma_{th}$ (the second threshold value) set on the basis of the non-negative-valued averages $\mu_1$ to $\mu_4$, in a period immediately after the system is started up. Then, if the subject S is determined as with no body motion, then the system calculates the average amplitude $A_{AVn}$ of the respiratory waveform BW of the subject S and, thereafter, determines the body motion of the subject S on the basis of a comparison between the threshold value $\sigma s_{TH}$ (the first threshold value) and the simple average $\sigma s_{AV}$ of the normalized standard deviations $\sigma s_1$ to $\sigma s_4$ which are normalized by the average amplitude $A_{AVn}$.

INDUSTRIAL APPLICABILITY

According to the body motion determining system of the present invention, it is possible to determine whether or not the subject has a body motion with high precision by reducing the influence of the subject's frame and respiratory depth. Therefore, by using the body motion determining system of the present invention, it is possible to provide high-quality medical treatments and caregiving services on the basis of those high-precision determinations.

PARTS LIST

1: load detecting unit, 11, 12, 13, 14: load detector, 2: A/D converting unit, 3: control unit, 31: standard deviation calculating unit, 32: respiratory waveform drawing unit, 33, 34: body motion determining unit, 35: respiratory rate calculating unit, 4: storage unit, 5: display unit, 6: notifying unit, 7: input unit, 100: body motion determining system, 200: biological state monitoring system, BD: bed, BDS: bed system, S: subject.

**Claims**

1. A body motion determining system (100, 200) configured to determine whether or not a subject (S) on a bed (BD) has a body motion, the system (100, 200) comprising:

   a plurality of load detectors (11-14) each configured to detect a load of the subject (S) on the bed (BD);
   a respiratory waveform obtaining unit (32) configured to obtain a respiratory waveform (BW) of the subject (S) based on a temporal variation of the load of the subject (S) detected by each of the plurality of load detectors (11-14); and
   a body motion determining unit (33, 34) configured to determine whether or not the subject (S) has the body motion, based on a comparison between a first threshold value and a standard deviation of the temporal variation of the load of the subject (S) detected by at least one of the plurality of load detectors (11-14),

wherein the body motion determining unit (33, 34) is configured to compensate the standard deviation to be used in the comparison by an amplitude of the respiratory waveform (BW), and

the compensating is performed by dividing the standard deviation by the amplitude of the respiratory waveform (BW).

2. The body motion determining system (100, 200) according to claim 1, wherein the body motion determining unit (33, 34) is configured to compensate the standard deviation to be used in the comparison by a latest amplitude of the respiratory waveform (BW), the latest amplitude being obtained based on a latest peak of the respiratory waveform (BW) which is the latest at a point of time of determining whether or not the subject (S) has a body motion, and a peak directly preceding the latest peak.

3. The body motion determining system (100, 200) according to claim 2, wherein the body motion determining unit (33, 34) is configured to compensate the standard deviation to be used in the comparison by an average value of the latest amplitude of the respiratory waveform (BW) and at least one amplitude of the respiratory waveform (BW) in a period directly preceding a point of time of obtaining the latest amplitude.

4. The body motion determining system (100, 200) according to claim 2 or 3, wherein the body motion determining unit (33, 34) is configured to continue, after a determination that the subject (S) has the body motion, to compensate the standard deviation using the latest amplitude at a point of time of the determination that the subject (S) has the body motion.

5. The body motion determining system (100, 200) according to claim 3, wherein the plurality of load detectors (11-14) include a first load detector (11), a second load detector (12), a third load detector (13), and a fourth load detector (14); and

the determining of whether or not the subject (S) has the body motion based on the comparison between the first threshold value and the standard deviation performed by the body motion determining unit (33, 34) is determining whether or not the subject (S) has the body motion based on a comparison between the first threshold value and a simple average of $\sigma_1/A_{AVn}$, $\sigma_2/A_{AVn}$, $\sigma_3/A_{AVn}$, and $\sigma_4/A_{AVn}$, provided that $\sigma_1$ is a first standard deviation of the temporal variation of the load of the subject (S) detected by the first detector (11), $\sigma_2$ is a second standard deviation of the temporal variation of the load of the subject (S) detected by the second detector (12), $\sigma_3$ is a third standard deviation of the temporal variation of the load of the subject (S) detected by the third detector (13), $\sigma_4$ is a fourth standard deviation of the temporal variation of the load of the subject (S) detected by the fourth detector (14), and $A_{AVn}$ is the average value.

6. The body motion determining system (100, 200) according to any one of claims 1 to 5, wherein the body motion determining unit (33, 34) is configured to determine a period during which the standard deviation is equal to or lower than a predetermined value as a resting period in which the subject (S) merely performs a respiration, and compensate the standard deviation to be used in the comparison by the amplitude of the respiratory waveform (BW) in the resting period.

7. The body motion determining system (100, 200) according to any one of claims 1 to 5, further comprising:

a non-negative-valued average calculating unit (343) configured to calculate a non-negative-valued average of a detection value of at least one of the load detectors (11-14); and

a threshold value setting unit (345) configured to set a second threshold value to be used to determine whether or not the subject (S) has a body motion, based on the non-negative-valued average calculated in the resting period in which the subject (S) merely performs the respiration,

wherein the body motion determining unit (33, 34) is configured to perform a determination to determine whether or not the subject (S) has the body motion based on a comparison between the second threshold value and the standard deviation, and then, after determining that the subject (S) has no body motion by the determination based on the comparison between the second threshold value and the standard deviation, perform a determination to determine whether or not the subject (S) has the body motion based on the comparison between the first threshold value and the standard deviation; and

the body motion determining unit (33, 34) is configured to compensate the standard deviation to be used in the comparison with the first threshold value by the amplitude of the respiratory waveform (BW) obtained in a period which is determined, by the determination based on the comparison between the second threshold value and the standard deviation, as a period in which the subject (S) has no body motion.

8. A bed system (BDS) comprising:

a bed (BD); and
the body motion determining system (100, 200) as defined in any one of claims 1 to 7.

**Patentansprüche**

1. Körperbewegungsbestimmungssystem (100, 200), das konfiguriert ist, um zu bestimmen, ob ein Subjekt (S) auf einem Bett (BD) eine Körperbewegung aufweist oder nicht, das System (100, 200) umfassend:

eine Vielzahl von Lastdetektoren (11-14), die jeweils konfiguriert sind, um eine Last des Subjekts (S) auf dem Bett (BD) zu erfassen;
Atemwellenformerhaltungseinheit (32), die konfiguriert ist, um eine Atemwellenform (BW) des Subjekts (S) basierend auf einer zeitlichen Variation der Last des Subjekts (S) zu erhalten, die durch jeden der Vielzahl von Lastdetektoren (11-14) erfasst wird; und
eine Körperbewegungsbestimmungseinheit (33, 34), die konfiguriert ist, um zu bestimmen, ob das Subjekt (S) die Körperbewegung aufweist oder nicht, basierend auf einem Vergleich zwischen einem ersten Schwellenwert und einer Standardabweichung der zeitlichen Variation der Last des Subjekts (S), die durch mindestens einen der Vielzahl von Lastdetektoren (11-14) erfasst wird,
wobei die Körperbewegungsbestimmungseinheit (33, 34) konfiguriert ist, um die Standardabweichung, die in dem Vergleich verwendet werden soll, durch eine Amplitude der Atemwellenform (BW) auszugleichen, und das Ausgleichen durch Teilen der Standardabweichung durch die Amplitude der Atemwellenform (BW) durchgeführt wird.

2. Körperbewegungsbestimmungssystem (100, 200) nach Anspruch 1, wobei die Körperbewegungsbestimmungseinheit (33, 34) konfiguriert ist, um die Standardabweichung, die in dem Vergleich verwendet werden soll, durch eine letzte Amplitude der Atemwellenform (BW) auszugleichen, wobei die letzte Amplitude basierend auf einer letzten Spitze der Atemwellenform (BW), die zu einem Zeitpunkt des Bestimmens, ob das Subjekt (S) eine Körperbewegung aufweist oder nicht, die letzte ist, und einer Spitze erhalten wird, die der letzten Spitze direkt vorangeht.

3. Körperbewegungsbestimmungssystem (100, 200) nach Anspruch 2, wobei die Körperbewegungsbestimmungseinheit (33, 34) konfiguriert ist, um die Standardabweichung, die in dem Vergleich verwendet werden soll, durch einen Durchschnittswert der letzten Amplitude der Atemwellenform (BW) und mindestens eine Amplitude der Atemwellenform (BW) in einem Zeitraum auszugleichen, der einem Zeitpunkt des Erhaltens der letzten Amplitude direkt vorangeht.

4. Körperbewegungsbestimmungssystem (100, 200) nach Anspruch 2 oder 3, wobei die Körperbewegungsbestimmungseinheit (33, 34) konfiguriert ist, um nach einer Bestimmung, dass das Subjekt (S) die Körperbewegung aufweist, fortzufahren, um die Standardabweichung unter Verwendung der letzten Amplitude zu einem Zeitpunkt der Bestimmung, dass das Subjekt (S) die Körperbewegung aufweist, auszugleichen.

5. Körperbewegungsbestimmungssystem (100, 200) nach Anspruch 3, wobei die Vielzahl von Lastdetektoren (11-14) einen ersten Lastdetektor (11), einen zweiten Lastdetektor (12), einen dritten Lastdetektor (13) und einen vierten Lastdetektor (14) einschließt; und
das Bestimmen, ob das Subjekt (S) die Körperbewegung aufweist oder nicht, basierend auf dem Vergleich zwischen dem ersten Schwellenwert und der Standardabweichung, der durch die Körperbewegungsbestimmungseinheit (33, 34) durchgeführt wird, das Bestimmen, ob das Subjekt (S) die Körperbewegung aufweist oder nicht, basierend auf einem Vergleich zwischen dem ersten Schwellenwert und einem einfachen Durchschnitt von $\sigma_1/A_{AVn}$, $\sigma_2/A_{AVn}$, $\sigma_3/A_{AVn}$ und $\sigma_4/A_{AVn}$ ist, vorausgesetzt, dass $\sigma_1$ eine erste Standardabweichung der zeitlichen Variation der Last des Subjekts (S) ist, die durch den ersten Detektor (11) erfasst wird, $\sigma_2$ eine zweite Standardabweichung der zeitlichen Variation der Last des Subjekts (S) ist, die durch den zweiten Detektor (12) erfasst wird, $\sigma_3$ eine dritte Standardabweichung der zeitlichen Variation der Last des Subjekts (S) ist, die durch den dritten Detektor (13) erfasst wird, $\sigma_4$ eine vierte Standardabweichung der zeitlichen Variation der Last des Subjekts (S) ist, die durch den vierten Detektor (14) erfasst wird, und $A_{AVn}$ der Durchschnittswert ist.

**6.** Körperbewegungsbestimmungssystem (100, 200) nach einem der Ansprüche 1 bis 5, wobei die Körperbewegungsbestimmungseinheit (33, 34) konfiguriert ist, um einen Zeitraum, während dessen die Standardabweichung gleich oder niedriger als ein zuvor bestimmter Wert ist, als eine Ruheperiode zu bestimmen, in der das Subjekt (S) lediglich eine Atmung durchführt, und um die Standardabweichung, die in dem Vergleich verwendet werden soll, durch die Amplitude der Atemwellenform (BW) in der Ruheperiode auszugleichen.

**7.** Körperbewegungsbestimmungssystem (100, 200) nach einem der Ansprüche 1 bis 5, ferner umfassend:

eine nicht negativwertige durchschnittliche Recheneinheit (343), die konfiguriert ist, um einen nicht negativwertigen Durchschnitt eines Erfassungswerts von mindestens einem der Lastdetektoren (11- 14) zu berechnen; und eine Schwellenwerteinstelleinheit (345), die konfiguriert ist, um einen zweiten Schwellenwert einzustellen, der verwendet werden soll, um zu bestimmen, ob das Subjekt (S) eine Körperbewegung aufweist oder nicht, basierend auf dem nicht negativwertigen Durchschnitt, der in der Ruheperiode berechnet wird, in der das Subjekt (S) lediglich die Atmung durchführt, wobei die Körperbewegungsbestimmungseinheit (33, 34) konfiguriert ist, um eine Bestimmung, um zu bestimmen, ob das Subjekt (S) die Körperbewegung aufweist oder nicht, basierend auf einem Vergleich zwischen dem zweiten Schwellenwert und der Standardabweichung durchzuführen, und dann nach dem Bestimmen, dass das Subjekt (S) keine Körperbewegung aufweist, durch die Bestimmung basierend auf dem Vergleich zwischen dem zweiten Schwellenwert und der Standardabweichung aufweist, eine Bestimmung, um zu bestimmen, ob das Subjekt (S) die Körperbewegung aufweist oder nicht, basierend auf dem Vergleich zwischen dem ersten Schwellenwert und der Standardabweichung durchzuführen; und die Körperbewegungsbestimmungseinheit (33, 34) konfiguriert ist, um die Standardabweichung, die in dem Vergleich mit dem ersten Schwellenwert verwendet werden soll, durch die Amplitude der Atemwellenform (BW) auszugleichen, die in einem Zeitraum erhalten wird, der durch die Bestimmung basierend auf dem Vergleich zwischen dem zweiten Schwellenwert und der Standardabweichung als ein Zeitraum bestimmt wird, in dem das Subjekt (S) keine Körperbewegung aufweist.

**8.** Bettsystem (BDS), umfassend:

ein Bett (BD); und das Körperbewegungsbestimmungssystem (100, 200) nach einem der Ansprüche 1 bis 7.

**Revendications**

**1.** Système de détermination de mouvement corporel (100, 200) configuré pour déterminer si un sujet (S) sur un lit (BD) se déplace, le système (100, 200) comprenant :

une pluralité de détecteurs de charge (11-14) qui détectent chacun une charge du sujet (S) sur le lit (BD) ; une unité d'acquisition de forme d'onde respiratoire (32) configurée pour obtenir une forme d'onde respiratoire (BW) du sujet (S) sur la base d'une variation temporelle de la charge du sujet (S) détectée par chacun de la pluralité de détecteurs de charge (11-14) ; et une unité de détermination de mouvement corporel (33, 34) configurée pour déterminer si le sujet (S) se déplace, sur la base d'une comparaison entre une première valeur seuil et un écart-type de la variation temporelle de la charge du sujet (S) détectée par au moins l'un de la pluralité de détecteurs de charge (11-14), dans lequel l'unité de détermination de mouvement corporel (33, 34) est configurée pour compenser l'écart-type à utiliser dans la comparaison par une amplitude de la forme d'onde respiratoire (BW), et la compensation est effectuée en divisant l'écart-type par l'amplitude de la forme d'onde respiratoire (BW).

**2.** Système de détermination de mouvement corporel (100, 200) selon la revendication 1, dans lequel l'unité de détermination de mouvement corporel (33, 34) est configurée pour compenser l'écart-type à utiliser dans la comparaison par une amplitude la plus récente de la forme d'onde respiratoire (BW), l'amplitude la plus récente étant obtenue sur la base d'une crête la plus récente de la forme d'onde respiratoire (BW) qui est la plus récente au moment de déterminer si le sujet (S) se déplace, et une crête précédant directement la dernière crête.

**3.** Système de détermination de mouvement corporel (100, 200) selon la revendication 2, dans lequel l'unité de détermination de mouvement corporel (33, 34) est configurée pour compenser l'écart-type à utiliser dans la comparaison par une valeur moyenne de l'amplitude la plus récente de la forme d'onde respiratoire (BW) et au moins une amplitude de la forme d'onde respiratoire (BW) dans un laps de temps précédant directement un moment d'obtention de la dernière amplitude.

4. Système de détermination de mouvement corporel (100, 200) selon la revendication 2 ou 3, dans lequel l'unité de détermination de mouvement corporel (33, 34) est configurée pour continuer, après une détermination que le sujet (S) se déplace, pour compenser l'écart-type à l'aide de la dernière amplitude au moment de déterminer que le sujet (S) se déplace.

5. Système de détermination de mouvement corporel (100, 200) selon la revendication 3, dans lequel la pluralité de détecteurs de charge (11-14) inclut un premier détecteur de charge (11), un deuxième détecteur de charge (12), un troisième détecteur de charge (13) et un quatrième détecteur de charge (14) ; et
la détermination du fait de savoir si le sujet (S) se déplace sur la base de la comparaison entre la première valeur seuil et l'écart type effectué par l'unité de détermination de mouvement corporel (33, 34) consiste à déterminer si le sujet (S) se déplace sur la base d'une comparaison entre la première valeur seuil et une moyenne simple de $\sigma_1/A_{AVn}$, $\sigma2/A_{AVn}$, $\sigma3/A_{AVn}$, et $\sigma4/A_{AVn}$, à condition que $\sigma_1$ est un premier écart-type de la variation temporelle de la charge du sujet (S) détectée par le premier détecteur (11), $\sigma_2$ est un deuxième écart-type de la variation temporelle de la charge du sujet (S) détectée par le deuxième détecteur (12), $\sigma_3$ est un troisième écart-type de la variation temporelle de la charge du sujet (S) détectée par le troisième détecteur (13), $\sigma_4$ est un quatrième écart-type de la variation temporelle de la charge du sujet (S) détectée par le quatrième détecteur (14), et AAVn est la valeur moyenne.

6. Système de détermination de mouvement corporel (100, 200) selon l'une quelconque des revendications 1 à 5, dans lequel l'unité de détermination de mouvement corporel (33, 34) est configurée pour déterminer un laps de temps pendant lequel l'écart-type est égal ou inférieur à une valeur prédéterminée en tant que période de repos pendant laquelle le sujet (S) effectue simplement une respiration, et pour compenser l'écart-type à utiliser dans la comparaison par l'amplitude de la forme d'onde respiratoire (BW) pendant la période de repos.

7. Système de détermination de mouvement corporel (100, 200) selon l'une quelconque des revendications 1 à 5, comprenant en outre :

une unité de calcul de moyenne non négative (343) configurée pour calculer une moyenne non négative d'une valeur de détection d'au moins un des détecteurs de charge (11-14) ; et
une unité de définition de valeur seuil (345) configurée pour définir une seconde valeur seuil à utiliser pour déterminer si le sujet (S) se déplace, sur la base de la moyenne non négative calculée dans la période de repos pendant laquelle le sujet (S) effectue simplement la respiration,
dans lequel l'unité de détermination de mouvement corporel (33, 34) est configurée pour effectuer une détermination pour déterminer si le sujet (S) se déplace sur la base d'une comparaison entre la seconde valeur seuil et l'écart-type, puis, après avoir déterminé que le sujet (S) ne se déplace pas grâce à la détermination sur la base de la comparaison entre la seconde valeur seuil et l'écart-type, effectuer une détermination pour déterminer si le sujet (S) se déplace sur la base de la comparaison entre la première valeur seuil et l'écart-type ; et
l'unité de détermination de mouvement corporel (33, 34) est configurée pour compenser l'écart-type à utiliser dans la comparaison avec la première valeur seuil par l'amplitude de la forme d'onde respiratoire (BW) obtenue pendant un laps de temps qui est déterminé, par la détermination sur la base de la comparaison entre la seconde valeur seuil et l'écart-type, en tant que laps de temps pendant lequel le sujet (S) ne se déplace pas.

8. Système de lit (BDS) comprenant :

un lit (BD) ; et
le système de détermination de mouvement corporel (100, 200) tel que défini selon l'une quelconque des revendications 1 à 7.

Fig. 1

**Fig. 2**

| LOAD DETECTING STEP | S11 |
| STANDARD DEVIATION CALCULATING STEP | S12 |
| RESPIRATORY WAVEFORM DRAWING STEP | S13 |
| BODY MOTION DETERMINING STEP | S14 |
| DISPLAY STEP | S15 |

**Fig. 3**

Fig. 4

(a)

(b)

Fig. 5

$\sigma s_{AV}(A_{AV2}) < \sigma s_{TH}$
WITHOUT
BODY MOTION

$\sigma s_{AV}(A_{AV1})$
$< \sigma s_{TH}$

$\sigma s_{AV}(A_{AV2})$
$\geqq \sigma s_{TH}$

$\sigma s_{AV}(A_{AV2})$
$< \sigma s_{TH}$

WITHOUT
BODY
MOTION

WITHOUT
BODY
MOTION

WITH
BODY
MOTION

WITHOUT
BODY
MOTION

t[s]

$t_1$          $t_2$          $t_{21}$          $t_{22}$

$p_0$          $p_1$          $p_2$          $p_3$

BW

$A_1$          $A_2$

Fig. 6

Fig. 7

```
┌─────────────────────────┐
│   LOAD DETECTING STEP    │────── S21
└─────────────────────────┘
            │
            ▼
┌─────────────────────────┐
│      BODY MOTION         │────── S22
│   DETERMINING STEP       │
└─────────────────────────┘
            │
            ▼
┌─────────────────────────┐
│   RESPIRATORY RATE       │────── S23
│   CALCULATING STEP       │
└─────────────────────────┘
            │
            ▼
┌─────────────────────────┐
│      DISPLAY STEP        │────── S24
└─────────────────────────┘
```

**Fig. 8**

34

```
┌─────────────────────────────────────────────────┐
│                                                   │
│  ┌─────────────────────────────────────────┐     │
│  │   DC COMPONENT ELIMINATING UNIT          │─── 341
│  └─────────────────────────────────────────┘     │
│                                                   │
│  ┌─────────────────────────────────────────┐     │
│  │  STANDARD DEVIATION CALCULATING UNIT     │─── 342
│  └─────────────────────────────────────────┘     │
│                                                   │
│  ┌─────────────────────────────────────────┐     │
│  │    NON-NEGATIVE-VALUED AVERAGE           │─── 343
│  │         CALCULATING UNIT                 │     │
│  └─────────────────────────────────────────┘     │
│                                                   │
│  ┌─────────────────────────────────────────┐     │
│  │   RESTING PERIOD DETERMINING UNIT        │─── 344
│  └─────────────────────────────────────────┘     │
│                                                   │
│  ┌─────────────────────────────────────────┐     │
│  │    THRESHOLD VALUE SETTING UNIT          │─── 345
│  └─────────────────────────────────────────┘     │
│                                                   │
│  ┌─────────────────────────────────────────┐     │
│  │         DETERMINING UNIT                 │─── 346
│  └─────────────────────────────────────────┘     │
│                                                   │
└─────────────────────────────────────────────────┘
```

**Fig. 9**

```
┌─────────────────────────┐
│      DC COMPONENT       │ ～ S221
│    ELIMINATING STEP     │
└─────────────────────────┘
            │
            ▼
┌─────────────────────────┐
│     RESTING PERIOD      │ ～ S222
│    DETERMINING STEP     │
└─────────────────────────┘
            │
            ▼
┌─────────────────────────┐
│     THRESHOLD VALUE     │ ～ S223
│      SETTING STEP       │
└─────────────────────────┘
            │
            ▼
┌─────────────────────────┐
│    DETERMINING STEP     │ ～ S224
└─────────────────────────┘
```

**Fig. 10**

Fig. 11

Fig. 12

Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060169282 A1
- JP 2016209327 A
- WO 2017056476 A1
- JP 2014195543 A
- JP 5998559 B
- JP 4829020 B
- JP 4002905 B
- JP 6105703 B